# EUROPEAN PATENT APPLICATION

(11) **EP 4 095 248 A1**
(43) Date of publication of application: **30.11.2022**
(21) Application number: 21175600.2
(22) Date of filing: 25.05.2021
(51) Int. Cl.: C12N 15/82, C12N 15/113, A61K 31/713

(54) **CIRCRNAS FOR GENE SILENCING**

(71) Applicant: Max-Planck-Gesellschaft zur Förderung der Wissenschaften e.V., 80539 München (DE)
(72) Inventor: CARROLL, Bernard John, Queensland, 4072 (AU); KRAGLER, Dr. Friedrich, 14467 Potsdam (DE); KADAM, Dr. Uhlas, 431536 Parbhani (IN)
(74) Representative: Wolff, Kerstin Andrea

(57) **Abstract**

The present invention relates to the field of gene silencing. The present invention *inter alia* concerns circular RNAs, compositions and kits comprising circular RNAs, methods of producing circular RNAs, methods of inhibiting the expression of a target gene or the function of a target gene in a cell, and uses of circular RNAs and compositions comprising circular RNAs.

## Description

### Field of the invention

The present invention relates to the field of gene silencing. The present invention *inter alia* concerns circular RNAs, compositions and kits comprising circular RNAs, methods of producing circular RNAs, methods of inhibiting the expression of a target gene or the function of a target gene in a cell, and uses of circular RNAs and compositions comprising circular RNAs.

### Background of the invention

The discovery that gene silencing is induced by double-stranded RNA (Fire et al., 1998, Nature 391, p.806-811) enabled researchers to express inverted repeat RNAs (dsRNA/invRNA) encoded by transgenes to suppress gene activity in a homology-dependent manner (Waterhouse et al., 1998, Proc Natl Acad Sci U S A 95, p. 13959-13964; Waterhouse et al., 1999, Trends Plant Sci 4, p.452-457; Carrington, 2000, Nature 408, p.150-151; Carrington et al., 2001, Virology 281, p.1-5; Waterhouse et al., 2001, Trends Plant Sci 6, p.297-301; Fusaro et al., 2006, EMBO Rep 7, p.1168-1175; Borges and Martienssen, 2015, Nat Rev Mol Cell Biol 16, p.727-741; Mitter et al., 2017, Nat Plants 3, 16207). It is now well established that dsRNA is processed by DICER-like (DCL) proteins into small regulatory RNAs (sRNAs) that guide silencing of endogenous genes and viruses at the transcriptional and post-transcriptional level (Angell and Baulcombe, 1997, EMBO J 16, p. 3675-3684; Ratcliff et al., 1997, Science 276, p.1558-1560; Baulcombe, 1999, Curr Opin Plant Biol 2, p.109-113). Remarkably, once gene silencing is initiated in a cell of multicellular plants and many multicellular animal species, the silencing can spread systemically throughout the entire organism. In plants, systemic spreading of gene silencing plays a key evolutionary role in recovery to virus infection and in viral defense in general (Angell and Baulcombe, 1997, EMBO J 16, p. 3675-3684; Ratcliff et al., 1997, Science 276, p.1558-1560; Baulcombe, 2004, Curr Opin Plant Biol 2, p.109-113). Systemic spreading of gene silencing can also be demonstrated in plants by grafting of transgenic lines carrying silenced transgenes onto lines that express an homologous transgene, agro-infiltration and transient expression of transgenes in tobacco leaves, particle bombardment of transgenes, or by using recombinant viruses to systemically silence endogenous genes (i.e. virus-induced gene silencing, VIGS) (Palauqui et al., 1997, EMBO J 16, p.4738-4745; Voinnet and Baulcombe, Nature 389, p.553, 1997; Yoo et al., 2004, Plant Cell 16, p.1979-2000).

In addition to conferring resistance to homologous viruses, inverted repeat transgenes can also be designed to successfully target homologous endogenous genes in fungal pathogens (Weiberg et al., 2013, Science 342, p.118-123; Zhang et al., 2016, Mol Plant 9, p.939-942) and insect pests of plants (Gordon and Waterhouse, 2007, Nat Biotechnol 25, p.1231-1232; Lilley et al., 2012, Parasitology 139, p.630-640; Koch et al., 2013, J Cell Sci 123, p.3389-3400). Furthermore, exogenous applications of in vitro synthesized dsRNA have also been shown to induce gene silencing of homologous viruses, transgenes, fungal pathogens, and insect pests of plants (see references in Mitter et al., 2017, Nat Plants 3, 16207). Topically applied dsRNA is most likely taken up by plant, fungal, and insect cells before it is processed by DCL endonucleases into siRNAs, which are then incorporated into the RNA-induced silencing complex (RISC) to guide RNA interference (RNAi). However, the uptake of exogenous dsRNA into plant cells and tissues is poorly understood.

A primary barrier for entry of exogenous applied biomolecules into plant cells is obviously its cell wall, which is a cellulosic mesh of at least 100 nm thick and with a pore size of <15 nm. Such a matrix limits cellular uptake of large RNAs molecules. Clay nanosheets are known to stabilize dsRNA applied to plants and despite the presence of the cell wall, facilitate the uptake of dsRNA and induction of virus resistance in plants (Mitter et al., 2017, Nat Plants 3, 16207). In these experiments, relatively high amounts of linear dsRNA in the range of several µg / mm² plant surface were used in complex with clay nanosheets in a formulation known as BioClay (Mitter et al., 2017, Nat Plants 3, 16207). To enhance the delivery of exogenous RNA into plant cells, other approaches involving conjugation chemistry or carrier compounds has been tested (Jiang et al., 2014, Nanoscale 6, p.9965-9969; Numata et al., 2014, Plant Biotechnol J 12, p.1027-1034; Takekata et al., 2014, J Insect Physiol 68, p.16-22). Nanocarriers such as tetrahedron, 1D hairpin tiles, and 1D nanostring have been used to improve the delivery of small RNAs into tobacco cells (Xue et al., 2019, Chem Commun (Camb) 55, p.4222-4225; Zhang et al., 2019, Anal Chem 91, p.7086-7096). However, these delivery-enhancing modifications have several drawbacks including instability, cost, and lack of robust technical reproducibility in chemical synthesis. To circumvent the need for carrier compounds, alternative high-pressure delivery methods such as biolistic bombardment and air brush applications were implemented and significantly improved the rate of gene silencing induced by topical application of dsRNA (Dalakouras et al., 2016, Plant J 87, p.202-214; Dalakouras et al., 2016, Front Plant Sci 7, 1327; Dubrovina et al., 2019, Int J Mol Sci 20; Dubrovina and Kiselev, 2019, Int J Mol Sci 20). Although these high-pressure application methods are effective in RNA delivery, they cause mechanical damage to plant tissues and adversely affect plant growth and increase pathogen susceptibility.

Accordingly, there is a need for improved dsRNAs and methods of gene silencing in eukaryotes, such as animals and plants. In particular, dsRNAs that are cost-efficient and effective at low concentrations, and/or that can be easily taken up by target eukaryotic cells, e.g. plant cells, are needed.

### Summary of the invention

The present invention solves the above need by *inter alia* providing new forms of dsRNA with the capacity to enhance the induction of gene silencing when applied to eukaryotic cells and organisms, e.g. plants. In particular, the inventors included structural features to protect the dsRNA from exonucleases and/or that are known to be necessary for long-distance mRNA transport over graft junctions in plants. These structural modifications involved circularizing the dsRNA (circRNAs), adding tRNA-like sequence (TLS) motifs, and incorporating methylated cytosine bases (m5C) into the dsRNA. Both TLSs and m5C have been recently shown to enhance long-distance transport of mRNAs across graft junctions in plants (Zhang et al., 2016, Plant Cell 28, p.1237-1249; Yang et al., 2019, Curr Biol 29, 2465-2476.e2465). The inventors surprisingly found that such circular RNAs are highly efficient at inducing gene silencing.

Specifically, the inventors found topically applied circular RNAs are the most efficient known form of RNA for silencing both an endogenous gene and transgene in *Arabidopsis* (see Examples). Furthermore, the inventors show a significant increase in the efficiency of induction of gene silencing with incorporation of m⁵C into the circular RNAs (see Examples). Induction of gene silencing by topical application of circRNAs was achieved without the aid of carriers, mechanical delivery devices, or physical wounding of the plants. The introduced structural modifications in form of adding nicked or covalently closed (single chain) circular RNA and TLS motifs and the substitution of cytosine ribonucleotides with m⁵C ribonucleotides increased significantly the silencing efficiency. This increase might be attributed to several features of the designed circRNA constructs: i) improved stability of the topically applied circRNA molecules (see Examples), ii) enhanced circRNA processing to siRNAs, and iii) upon uptake of the RNA, improved RNA transport to distant cells mediated by TLS motifs and m⁵C modifications.

In a known non-transgenic approach, gene silencing is induced by relative high concentrations of topically applied dsRNAs that have to be taken up by cells, perhaps transmitted systemically, and then processed to siRNAs to induce gene silencing. The inventors show that circularizing dsRNA and incorporation m⁵C and TLS motifs enhances both RNA stability and its capacity to induce gene silencing when applied topically onto plants (see Examples). According to reports of induction of gene silencing by topical applied siRNAs, the use of small 21 nt dsRNAs combined with pressure delivery appears to be the most effective way to induce RNA silencing in plants (Dalakouras et al., 2020, Plant Physiol 182, 38-50). In this approach, high-pressure spraying (at 7-8 bar pressure) of 1000 pmol of 21-, 22-, and 24-nt siRNAs efficiently induced systemic GFP silencing in transgenic N. *benthamiana* plants after three weeks (Dalakouras et al., 2016, Front Plant Sci 7, 1327). This relatively high efficiency is most likely achieved by the wounding the tissue, thereby increasing the uptake of RNA molecules. Supporting this interpretation, abrasive co-incubation of pepper mild mottle virus (PMMoV), tobacco etch virus (TEV), and alfalfa mosaic virus (AMV) with equal amounts of long homologous dsRNA (62 pmol) matching viral sequences induced silencing of the specific virus (Tenllado and Diaz-Ruiz, 2001, J Virol 75, p.12288-12297). The inventors applied approx. 8 pmol of circular RNAs of the invention for 48 hours to plants germinated in liquid growth medium. Although they did not apply the circRNA with high pressure spray devices, or use abrasion techniques to enhance RNA uptake, the circRNA was approximately 100 times more effective (see Examples) than that reported with high pressure spray experiments (Dalakouras et al., 2016, Front Plant Sci 7, 1327).

An alternative approach used to induce silencing was to infiltrate small dsRNA - peptide complexes into fully expanded Arabidopsis or poplar leaves (Numata et al., 2014, Plant Biotechnol J 12, p.1027-1034). In this report, topical application of approximately 20 pmol of 21-nt siRNAs in complex with a peptide resulted in transient down-regulation of a YFP transgene but not the endogenous CHALCONE SYNTHASE gene. However, the Numata et al. (2014) approach failed to detect sustained silencing and induce systemic spread of gene silencing, which is in contrast to the results obtained with circular RNAs of the present invention (see Examples). Using 8 pmol of a circular RNA with two simple loops (circRNA-2xHP) or a combination of one simple loop with dDT or a full TLS (circRNA-dDT/TLS), the inventors were able to demonstrate a high frequency of triggering systemic silencing of a *YFP*/*GFP* transgene (see Examples) and the *STM* endogenous gene (see Examples) in Arabidopsis. Expression of the endogenous *STM* gene in non-transgenic wild-type plants is specifically expressed in shoot apical meristem (SAM) and not in cotyledons, roots or leaves. The results of the Examples thus suggest that topically applied *STM-*targeting circular RNAs of the invention are taken up directly into the SAM or systemically transmitted to the SAM following uptake by other cells in order to induce silencing of *STM.*

In summary, covalently closing dsRNA molecules (thereby producing single chain circular RNAs of the invention), as well as incorporating TLS motifs and m⁵C nucleotides into the circular RNAs of the invention, enhances their potency as topically applied RNA molecules for inducing gene silencing.

In the following, the aspects of the invention are described. Embodiments of these aspects are also mentioned.

### First aspect: circular RNA

In an embodiment of a first aspect, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

In an embodiment, either the sense strand sequence or the antisense strand sequence contains a nick.

In a second embodiment of the first aspect, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

In an embodiment, in any of the above embodiments, the second loop sequence or the loop sequence of the second single-chain RNA molecule is a stem-loop sequence and comprises a tRNA-like sequence (TLS) motif structure, optionally wherein the first loop sequence orthe loop sequence of the first single-chain RNA molecule is also a stem-loop sequence and comprises a tRNA-like sequence (TLS) motif structure. In an embodiment, the TLS motif structure is
c. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
d. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
e. a viral 3' TLS sequences from a virus forming a tRNA related structure.
f. a viroid forming stem-loop or pseudo-knot structures related to tRNAs
g. a precursor micro RNA or messenger RNA stem-loop structure mediating intercellular RNA transport.

In an embodiment, the circular RNA of any of the above embodiments comprises at least one methylated cytosine (m⁵C) residue, optionally wherein all cytosine residues of the circular RNA are m⁵C residues.

In an embodiment, in any of the above embodiments, the stem formed from the sense strand sequence and the antisense strand sequence is 19 base pairs or longer in length.

### Second aspect: composition comprising the circular RNA of the first aspect

In a second aspect, the present invention provides a composition comprising the circular RNA of any one of the embodiments of the first aspect.

In an embodiment, the composition is a pharmaceutical composition further comprising a pharmaceutically acceptable carrier.

In an embodiment, the composition of any one of the above embodiments further comprises
h. a permeability-enhancing agent, optionally wherein the permeability-enhancing agent is selected from the group consisting of a surfactant, and organic solvent, an aqueous solution of an organic solvent, an oxidizing agent, an acid, a base, an oil, an enzyme, or a combination thereof; or
i. an adjuvant; or
j. a peptide-carrier; or
k. a vesicular endocytosis-carrier; or
I. a micro-carrier; or
m. a nano -carrier.

### Third aspect: kit comprising the circular RNA of the first aspect or the compositions of the second aspect

In a third aspect, the present invention provides a kit comprising the circular RNA of any one of the embodiments of the first aspect or the composition of any one of the embodiments of the second aspect.

### Fourth aspect: method of producing the circular RNA of the first aspect

In an embodiment of a fourth aspect, the present invention provides a method of producing a circular RNA, comprising the steps of:
a. synthesizing a first single-chain RNA molecule comprising:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, optionally wherein the loop sequence is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem;
b. synthesizing a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, optionally wherein the loop sequence is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem;
c. hybridizing the first and second single-chain RNA molecules such that the sense and antisense strand sequences pair to form a stem, thereby producing a double-chain circular RNA; and
d. optionally ligating the hybridized first and second single-chain RNA molecules of step c to form a single-chain circular RNA,
wherein the locations in the first and second single-chain RNA molecule are all 3' or all 5', wherein the loop sequence of the first and the loop sequence of the second single-chain RNA molecules have different or the same sequences, wherein the sense and antisense strand sequences correspond to nucleotide sequences of a target gene or target transcript, and wherein the nucleotide sequences of the target gene and or transcript are complementary to each other.

In another embodiment of the fourth aspect, the present invention provides a method of producing a circular RNA, comprising the steps of:
a. synthesizing a single-chain RNA molecule comprising:
   i. a sense strand sequence,
   ii. a first pairing sequence located 3' or 5' of the sense strand sequence,
   iii. a first loop sequence located 3' or 5' of the first pairing sequence, wherein the first loop sequence is at least 3 nucleotides in length, and
   iv. a second pairing sequence located 3' or 5' of the first loop sequence, wherein the second pairing sequence is complementary to the first pairing sequence,
   v. an antisense strand sequence complementary to the sense strand sequence, located 3' or 5' of the second pairing sequence; and
   vi. a loop sequence located 3' or 5' of the antisense strand sequence, optionally wherein the second loop sequence comprises a tRNA-like sequence (TLS) motif structure, and
   wherein the locations are all 3' or all 5',
b. hybridizing the single-chain RNA molecule such that the sense and antisense strand sequences pair to form a stem and the first and second pairing sequences from a stem, thereby producing a circular single-chain RNA; and
c. optionally ligating the hybridized circular single-chain RNA molecules of step b,
wherein the first and second loop sequences have different or the same sequences, wherein the sense and antisense strand sequences correspond to nucleotide sequences of a target gene or target transcript, and wherein the nucleotide sequences of the target gene and or transcript are complementary to each other.

In an embodiment, in anyone of the above methods, in steps a and b 5-methylcytidine-5'triphosphate is provided. In another embodiment, the circular RNA comprises a methylation motif and the method further comprises exposing the circular RNA to a methyltransferase enzyme modifying cytosine (C) to 5-methylcytosine (m5C) RNA in vivo, ex vivo, or in vitro.

### Fifth aspect: method of inhibiting the expression of a target gene or the function of a target transcript in a cell

In a fifth aspect, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other.

In an embodiment, the method is performed *in vitro, ex vivo,* or *in vivo.*

In an embodiment of any of the above methods of the fifth aspect, the cell is a cell selected from the group consisting of a eukaryotic cell, optionally wherein the cell is an animal cell, a fungal cell, or a plant cell.

In an embodiment of any of the above methods of the fifth aspect, the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant.

In an embodiment of any of the above methods of the fifth aspect, the cell is present in an organism, and the circular RNA or composition is introduced within a body cavity of the organism inside or outside the cell. In another embodiment, the cell is present in an organism, and wherein the circular RNA or composition is introduced into the organism by extracellular injection. In another embodiment, the cell is present in an organism, and the circular RNA or composition is introduced into the organism by feeding. In yet another embodiment, the cell is a plant cell present in a plant, and the circular RNA or composition is topically applied to the surface of the plant, optionally wherein the circular RNA or composition is applied by soaking, coating, spraying, biolistically introducing, electroporating or high-pressure spraying. In yet another embodiment, the cell is a plant cell present in a plant, and the circular RNA or composition is introduced into the plant by applying the circular RNA or composition on plant tissue after cutting the plant or on callus tissue formed after cutting the plant, optionally wherein the plant is a grape vine.

In an embodiment of any of the above methods of the fifth aspect, the expression of the target gene or function of the target transcript is inhibited by at least 10%, 15%, 20%, 25%, 30%, 35%, 40%, 45%, 50%, 55%, 60%, 65%, 70%, 75%, 80%, 85%, 90%, 95%, 96%, 97%, 98%, or 99%.

In an embodiment of any of the above methods of the fifth aspect, inhibiting the expression of a target gene or the function of a target transcript results in suppression of a pathogen. In another embodiment, the expression of the target gene or the function of the target transcript is dysregulated in the cell and inhibiting the expression of a target gene or function of the target transcript results in better regulation.

### Sixth aspect: circular RNA of the first aspect and composition of the second aspect for use

In a sixth aspect, the present invention provides the circular RNA of any one of the embodiments of the first aspect or the composition of any one of the embodiments of the second aspect for use in a method of treating a condition in a subject in need thereof, wherein the condition is selected from the group consisting of an infectious disease, feeding animals, a heritable disease, cancer, a disease involving the dysregulation of the expression of a gene, and a disease involving dysregulation of the function of a transcript.

### Brief description of the drawings

The figures shown in the following are merely illustrative and shall describe the present invention in a further way. These figures shall not be construed to limit the present invention thereto.

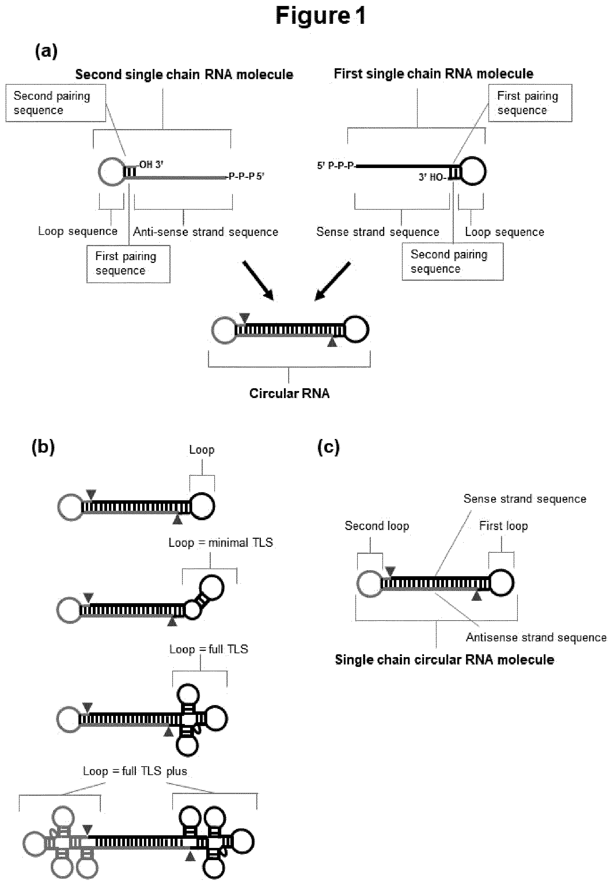

- **Figure 1**: Schematic structures of circular RNAs and single chain RNA molecules used to produce them. **(a)** single chain RNA molecules (top) produced by T7 synthesis containing a 5'-triphosphate cap (removable by e.g. alkalic phosphatase) are used to produce a circular RNA (bottom). Triangles indicate gaps between nucleic acid residues. **(b)** circular RNAs with two simple loops (top), one simple loop and one loop that is a minimal tRNA-like sequence (TLS) motif, also known as "dDT" (second from top), one simple loop and one loop that is a full TLS motif (second from bottom), or two full TLS motifs with an additional stem loop resulting from additional T7 sequences (bottom). **(c)** single chain circular RNA.

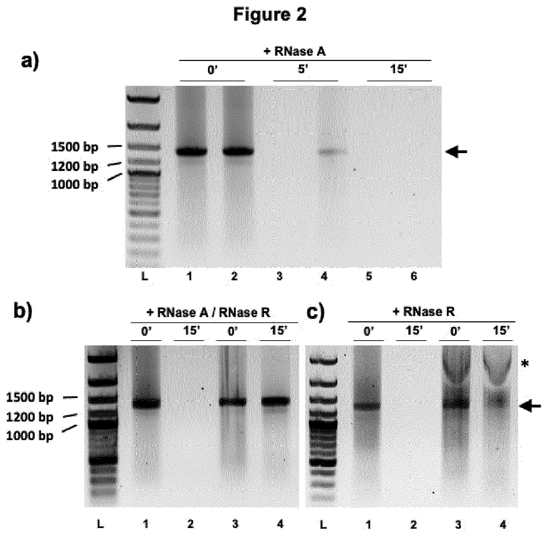

- **Figure 2**: Nuclease protection assays of second single chain RNA molecule with simple loop ("ssRNA_HP"), circular RNA comprising dDT ("nicked circRNA-dDT"), and single chain circular RNA comprising dDT ("ligated circRNA-dDT") directed to STM used in the Examples. **(a)** RNase A protection assay of ssRNA_HP (STM) compared to methylated m⁵C ssRNA_HP. Lanes: 1: ssRNA-HP (control), 2: ssRNA_HP-m5C (control), 3: ssRNA-C (+ RNase A, 5 min.), 4: ssRNA-HP m5C (+ RNase A, 5 min.), 5: ssRNA-HP (+ RNase A, 15 min.), 6: ssRNA-HP m5C (+ RNase A, RNAse R, 15 min.). **(b)** Quality control of nicked circRNA-dDT (STM) after annealing. Lanes: 1: ssRNA-HP (control), 2: ssRNA-HP (+ RNase A, RNase R, 15 min.), 3: circRNA-DT (control), 4: circRNA-DT (+ RNAse A, RNase R,15 min.). (c) Quality control of ligated circRNA-dDT (STM). Lanes: 1: ssRNA-HP (control), 2: ssRNA (+ RNase R, 15 min.), 3: circRNA-DT (control), 4: circRNA-DT (+RNase R,15 min.). The shifted band marked with the red asterisk corresponds to single-chain circRNA. L: 100 bp plus DNA Ladder.

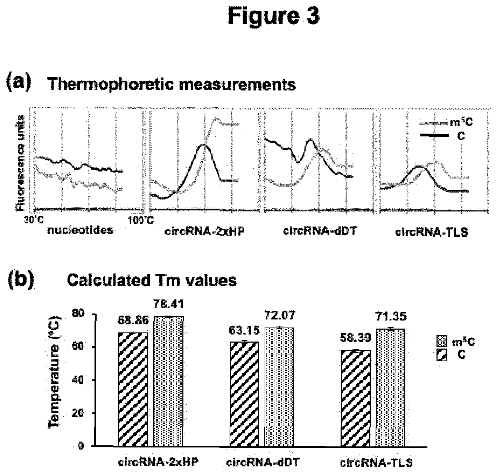

- **Figure 3**: Thermostability of single chain circRNA constructs. **(a)** Thermophoretic melting-curve analysis of non-methylated (C) and methylated (m⁵C) circRNA-2xHP, circRNA-dDT, and circRNA-TLS constructs. **(b)** Calculated Tₘ values of the circRNAs. Bars: Standard Deviation (S.D.).

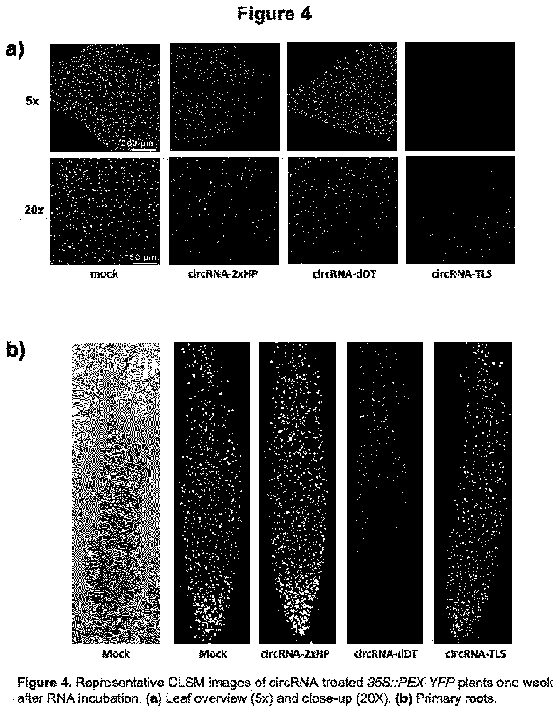

- **Figure 4**: Representative CLSM images of circRNA-treated *35S::PEX-YFP* plants one week after RNA incubation. **(a)** Leaf overview (5x) and close-up (20X). **(b)** Primary roots.

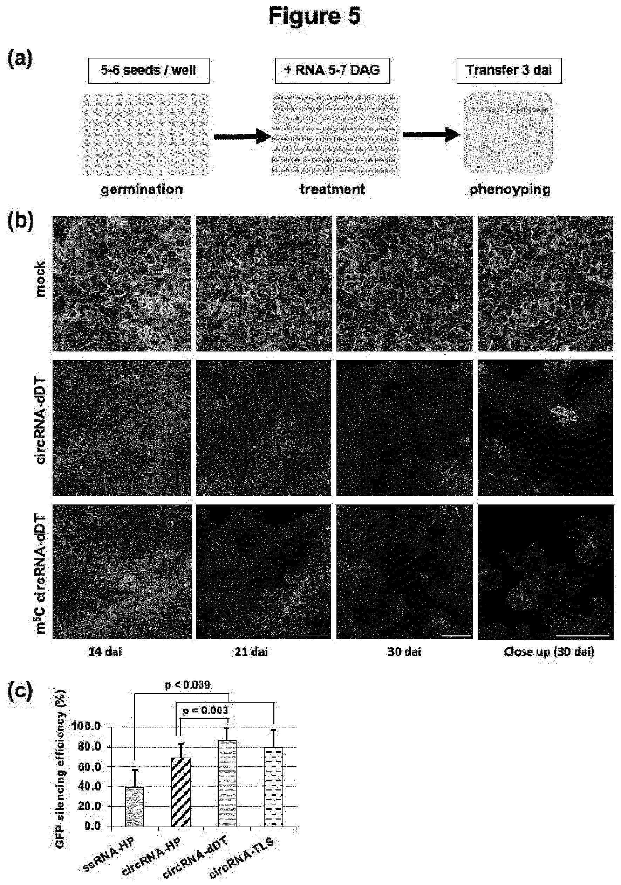

- **Figure 5**: Experimental design used for RNA incubation and evaluation of silencing by confocal microscopy (CLSM). **(a)** First 5-6 sterilized seeds were germinated per well (+ 100 µL ½ MS). Five to seven days after germination (DAG) 10 µl RNA (∼5µg) was added to each well and incubated for 3 days. Plants were transferred three days after incubation (dai) to square plates containing 0.5 MS agar (lacking RNA) and grown until further phenotyping. **(b)** CLSM images showing green fluorescence detected in leaves of mock and circRNA incubated *ER-GFP* plants. Note that a typical pattern of systemic *ER-GFP* silencing was exclusively detected in circRNA-treated plants at 14 dai, 20 dai, and 30 dai. Bar: 50µm. **(c)** ER-GFP gene silencing efficency (%) per construct category and significance of difference according to Students T-test; Experimental replicates: n>5; n> 38 plants per category.

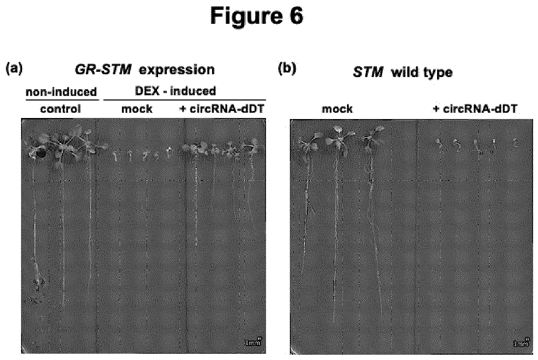

- **Figure 6**: Example of *STM* silencing phenotypes detected with *GR-STM* and wild-type plants incubated with circRNAs. **(a)** Effect of m⁵C circRNA-dDT (nicked) on growth of GR-STM transgenic plants. **(b)** Effect of m⁵C circRNA-dDT (nicked) on growth of wild-type plants.

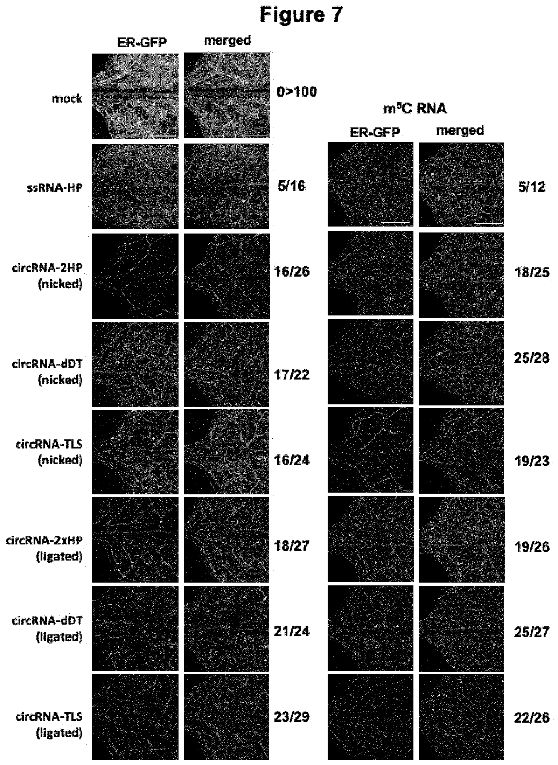

- **Figure 7**: Representative CLSM images of leaves from *ER-GFP* plants incubated with circRNAs showing gene silencing 14 days after incubation. Left panel: non-methylated, right panel: m5C methylated circRNAs. Numbers on the right indicate silenced plants vs. treated plants detected. Bar: 1000 µm.

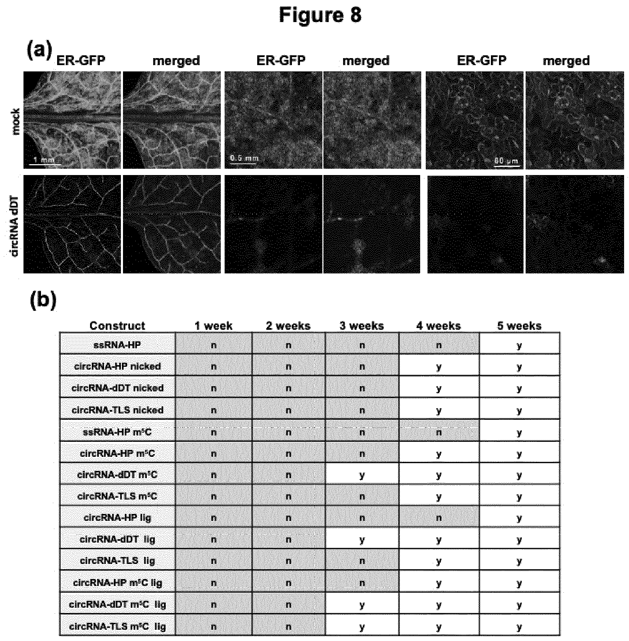

- **Figure 8**: Confocal images of leaves and epidermal cells from mock and circRNA-dDT (nicked) treated *ER-GFP* plants 14 days after incubation.

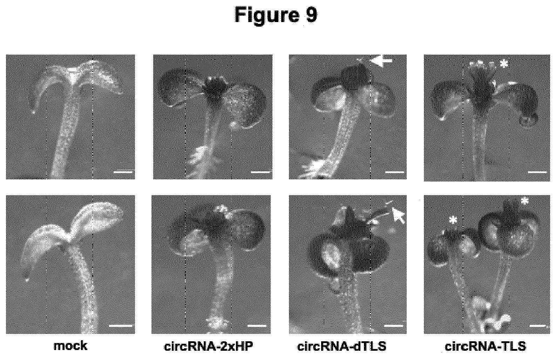

- **Figure 9**: Images of DEX-induced *GR-STM* plants incubated with circRNAs. Treatment with circRNA constructs allows growth progression of *GR-STM* plants and formation of leaves and flower-like structures 3 weeks after incubation. Note the progression of apical growth, formation of small leaves, trichomes appearing on leaves (arrows), and formation of flower-like structures (asterisks) after incubation with circRNA not detected with mock controls. Bar: 1mm.

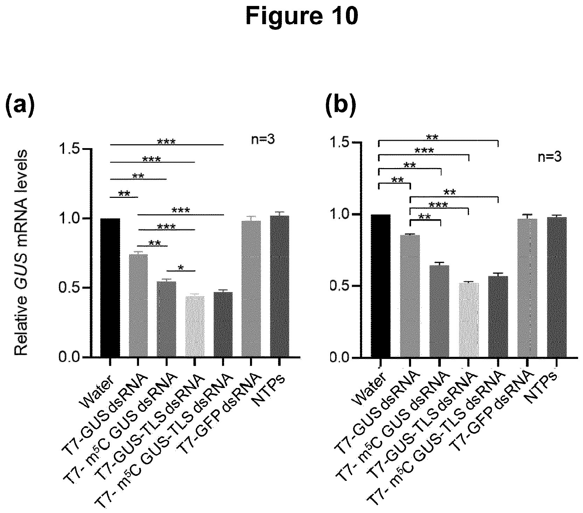

- **Figure 10**: Relative *GUS* mRNA measured by quantitative (q)RT PCR. **(a)** Controlled environmental chamber and **(b)** greenhouse grown *35S::GUS* transgenic *A. thaliana* seedlings (n=∼10) were incubated with 5µg methylated (m⁵C) and non-methylated *T7-GUS* ds RNA or *T7-GUS-TLS* ds RNA constructs after germination. RNA samples for qRT-PCR were harvested 5 days after incubation. Water, T7-GFPdsRNA, and NTPs treatments serve as controls. Unpaired t-tests were followed by Welch's correction, *P < 0.05, **P < 0.01 and ***P < 0.001. Error bars: mean SE.

### Definitions

For the sake of clarity and readability the following definitions are provided. Any technical feature mentioned for these definitions may be read on each and every embodiment of the invention. Additional definitions and explanations may be specifically provided in the context of these embodiments.

As used in the specification and the claims, the singular forms of "a" and "an" also include the corresponding plurals unless the context clearly dictates otherwise.

The term "about" in the context of the present invention denotes an interval of accuracy that a person skilled in the art will understand to still ensure the technical effect of the feature in question. The term typically indicates a deviation from the indicated numerical value of ±10% and preferably ±5%.

It needs to be understood that the term "comprising" is not limiting. For the purposes of the present invention, the term "consisting of" is considered to be a preferred embodiment of the term "comprising". If hereinafter a group is defined to comprise at least a certain number of embodiments, this is also meant to encompass a group which preferably consists of these embodiments only.

The term "nucleic acid" means any DNA or RNA molecule and is used synonymously with "polynucleotide". An "oligonucleotide" is a polynucleotide of a defined length, usually of a length of about 5 to about 1000 nucleotides, but not limited thereto. The specific order of the monomers, i.e. the order of the bases linked to the sugar/phosphate-backbone, is called the "nucleotide sequence".

The term "DNA" is the usual abbreviation for "deoxyribonucleic acid". A DNA molecule is a nucleic acid molecule, i.e. a polymer consisting of nucleotide monomers. These nucleotides are usually deoxy-adenosine-monophosphate, deoxy-thymidine-monophosphate, deoxy-guanosine-monophosphate and deoxy-cytidine-monophosphate monomers or analogs thereof which are - by themselves - composed of a sugar moiety (deoxyribose), a base moiety and a phosphate moiety, and polymerize by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone, is called the DNA sequence. DNA may be single stranded or double stranded. In the double stranded form, the nucleotides of the first strand typically hybridize with the nucleotides of the second strand, e.g. by A/T-base-pairing and G/C-base-pairing.

The term "RNA" is the usual abbreviation for ribonucleic acid. An RNA molecule is a nucleic acid molecule, i.e. a polymer consisting of nucleotide monomers, also called a chain. These nucleotides are usually adenosine-monophosphate, uridine-monophosphate, guanosine-monophosphate and cytidine-monophosphate monomers or analogs thereof, which are connected to each other along a so-called backbone. The backbone is formed by phosphodiester bonds between the sugar, i.e. ribose, of a first and a phosphate moiety of a second, adjacent monomer. The specific order of the monomers, i.e. the order of the bases linked to the sugar/phosphate-backbone, is called the RNA sequence. The term "RNA" generally refers to a molecule or to a molecule species selected from the group consisting of long-chain RNA, coding RNA, non-coding RNA, single stranded RNA (ssRNA), double stranded RNA (dsRNA), linear RNA (linRNA), circular RNA (circRNA), messenger RNA (mRNA), RNA oligonucleotides, small interfering RNA (siRNA), small hairpin RNA (shRNA), antisense RNA (asRNA), CRISPR/Cas9 guide RNAs, riboswitches, immunostimulating RNA (isRNA), ribozymes, aptamers, ribosomal RNA (rRNA), transfer RNA (tRNA), viral RNA (vRNA), retroviral RNA or replicon RNA, small nuclear RNA (snRNA), small nucleolar RNA (snoRNA), microRNA (miRNA), circular RNA (circRNA), and a Piwi-interacting RNA (piRNA).

Both DNA and RNA may also contain modified nucleotides. The term "modified nucleotides" as used herein will be recognized and understood by the person of ordinary skill in the art, and is for example intended to comprise nucleotides that comprise a modification. For example, any nucleotide different from G, C, U, T, A may be regarded as a "modified nucleotide". Modified nucleotides known in the art comprise 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl-inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-lodo-2'-deoxycytidine-5'-triphosphate, 5-iodouridine-5'-triphosphate, 5-lodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazoie-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate, xanthosine-5'-triphosphate. Particular preference is given to nucleotides for base modifications selected from the group of base-modified nucleotides consisting of 5-methylcytidine-5'-triphosphate, 7-deazaguanosine-5'-triphosphate, 5-bromocytidine-5'-triphosphate, and pseudouridine-5'-triphosphate, pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, and 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine, 5'-O-(1-thiophosphate)-adenosine, 5'-O-(1-thiophosphate)-cytidine, 5'-O-(1-thiophosphate)-guanosine, 5'-O-(1-thiophosphate)-uridine, 5'-O-(1-thiophosphate)-pseudouridine, 6-aza-cytidine, 2-thio-cytidine, alpha-thio-cytidine, Pseudo-iso-cytidine, 5-aminoallyl-uridine, 5-iodo-uridine, N1-methyl-pseudouridine, 5,6-dihydrouridine, alpha -thio-uridine, 4-thio-uridine, 6-aza-uridine, 5-hydroxy-uridine, deoxy-thymidine, 5-methyl-uridine, Pyrrolo-cytidine, inosine, alpha -thio-guanosine, 6-methyl-guanosine, 5-methyl-cytdine, 8-oxo-guanosine, 7-deaza-guanosine, N1-methyl-adenosine, 2-amino-6-Chloro-purine, N6-methyl-2-amino-purine, Pseudo-iso-cytidine, 6-Chloro-purine, N6-methyl-adenosine, alpha -thio-adenosine, 8-azido-adenosine, 7-deaza-adenosine, pseudouridine, N1-methylpseudouridine, N1-ethylpseudouridine, 2-thiouridine, 4'-thiouridine, 5-methyluridine, 2-thio-1-methyl-1-deaza-pseudouridine, 2-thio-1-methyl-pseudouridine, 2-thio-5-aza-uridine, 2-thio-dihydropseudouridine, 2-thio-dihydrouridine, 2-thio-pseudouridine, 4-methoxy-2-thio-pseudouridine, 4-methoxy-pseudouridine, 4-thio-1-methyl-pseudouridine, 4-thio-pseudouridine, 5-aza-uridine, dihydropseudouridine, 2'-0-methyl uridine, pseudouridine (ψ), N1-methylpseudouridine (m1ψ), 5-methylcytosine, and 5-methoxyuridine.

A "circular RNA" designates an RNA forming a circle. That is, a circular RNA does not contain unpaired, single-stranded ends of RNA molecules. A circular RNA may be made up of a single RNA chain (then termed a "single-chain circular RNA") that comprises no nicks, i.e. wherein the backbone of the chain is unbroken. An exemplary single chain RNA is depicted in Figure 1C. Alternatively, a single-chain circular RNA molecule may comprise a single nick, i.e. be one continuous ribonucleotide chain with one 3' end and one 5' end. In such a single-chain circular RNA, the nick must occur in a region that is hybridized, i.e. form a double stranded RNA structure (i.e. a stem), with another, complementary stretch of the same single-chain circular RNA to maintain circularity. A circular RNA may be made up of two separate RNA chains (then also referred to as a "double chain circular RNA"), with each chain being a continuous ribonucleotide chain with one 3' end and one 5' end. In such a double-chain circular RNA molecule, the two separate chains (i.e. single-chain RNA molecules) comprise sequences that are complementarity to a corresponding sequence on the respective other chain, such that they hybridize and form a double stranded RNA structure (i.e. a stem). The 3' and 5' ends of both chains of the double-chain circular RNA must be located within these paired sequences and be located immediately adjacent to each other (i.e. the 5' end of the first single chain RNA molecule is located directly next to the 3' end of the second single chain RNA molecule, and the 5' end of the second single chain RNA molecule is located directly next to the 3' end of the first single chain RNA molecule), such that there is no unpaired nucleotide residue on the complementary sequence. Such an arrangement is depicted in Figures 1A and 1B. Double-chain circular RNAs are also referred to as nicked circular RNAs herein.

A "nick" as used herein designates a gap in the backbone of an RNA between directly adjacent bases within a double-stranded RNA structure/stem.

A "sense strand sequence" as used herein refers to an RNA sequence that is complementary to an "antisense strand antisense", which is also an RNA sequence, and vice versa. The sense strand and the antisense strand sequences can be designed to suppress the function of a target gene, based on the nucleotide sequence of the target gene. That is, the sense and antisense strand sequences will also be complementary to nucleotide sequences within a target gene, which sequences are complementary to each other. Similarly, the sense and antisense strand sequences are complementary to corresponding nucleotide sequences within target transcripts resulting from transcription of the target gene. The designs can be confirmed by producing multiple sense and antisense strands and testing for each suppression efficiency. For example, designing using an algorithm for siRNA design or the like can be applied (Jaeger et al., Methods in Enzymology (1989) 183: p-281-306; Mathews et al., J. Mol. Biol. (1999) 288: p.911-940). When designing, it is preferable that the strands do not suppress the expression of genes other than a target gene, the genes having sequences similar to the target gene (which is known as the off target effect). The lengths of the sense and the antisense strands are preferably designed in the range of, for example, 19 to 31 bases, preferably 21 to 25 bases, more preferably 22 to 24 bases, and even more preferably 23 bases.

"Gene silencing" is the regulation of gene expression in a cell to prevent the expression of a target gene. Gene silencing can occur during either transcription or translation. That is, a "target gene" is a gene within a cell, i.e. a stretch of double stranded DNA within a cell that encodes for a gene product, which is to be silenced. If silencing occurs on the transcription level, the circular RNA of the invention will target the target gene by comprising sense and antisense strand sequences that are complementary to sequences within the target gene, which nucleotide sequences are in turn complementary to each other. Transcription of a "target gene" may give rise to a "target transcript". A "target transcript" is a transcript of a gene or target gene the translation or function of which is to be inhibited by the circular RNA of the invention. In such a case, the circular RNA will comprise sense and antisense strand sequences which are complementary to nucleotide sequences within the target transcript. Functions of target transcripts may include, but are not limited to, functioning as protein encoding messenger RNA (mRNA), ribosomal RNA (rRNA), transfer RNA (tRNA), small regulatory RNA (sRNA), small nuclear RNAs (snRNA), microRNAs (miRNAs), small interfering RNA (siRNAs), small nucleolar RNAs (snoRNAs), riboswitches, or catalytic RNAs. Due to the complementarity of the sense and antisense strand sequences to nucleotide sequences within the target DNA and/or target transcript, hybridization between them can occur.

A "loop sequence" as used herein is an RNA sequence comprising single stranded RNA sequences, i.e. sequences wherein no base-pairing occurs, located between RNA sequences which are base-paired to complementary RNA sequences to form double-stranded "stem" structures. A loop sequence may, e.g., be a "simple loop sequence", i.e. comprise no base-paired nucleotide residues at all. A loop sequence may also be more complex and comprise a "stem-loop" sequence, i.e. a sequence comprising one or more simple loops and stems. Such a stem-loop sequence may comprise a "tRNA-like sequence (TLS) motif structure". Exemplary loop sequence structures are indicated in Figures 1A, B, and C.

A "pairing sequence" as used herein refers to an RNA sequence that is complementary to another pairing sequence located within the same single-stranded RNA molecule, i.e. the same RNA chain, which is also an RNA sequence, such that they form a double-stranded RNA structure or stem. The lengths of the pairing sequence are preferably designed in the range of, for example, 3 to 15 bases, preferably 4 to 12 bases, more preferably 5 to 10 bases, and even more preferably 7 bases.

A "TLS motif structure" is a stem-loop structure that corresponds to or is derived from a naturally occurring transfer RNA ("tRNA"). Naturally occurring tRNAs include tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, with tRNA^{Met} being especially preferred. Naturally occurring tRNAs have a distinctive folded structure with three hairpin loops that form the shape of a three-leafed clover. One of these hairpin arms, called the "anticodon arm", contains a sequence called the anticodon in its loop, which can recognize and decode an mRNA codon. The other two hairpin structures are called the dihydrouridine arm or D-arm and the TψC arm. Additionally, there is a small "variable loop" located between the stems of the TψC arm and the anticodon arm. A TLS motif structure can comprise all of these structures of a naturally occurring tRNA ("full TLS"), or only parts thereof. A minimal TLS motif lacks the D and TψC arms and is also called "dDT" (Zhang et al., 2016, Mol Plant 9, p.939-942). Alternatively, a TLS may have the viral 3' TLS sequence from a virus forming a tRNA or pseudo-knot related structure (Colussi et al., Nature 2014 Jul 17; 511(7509): 366-369). Alternatively, a TLS may have the sequence of a viroid forming stem-loop or pseudo-knot structures related to tRNAs. Alternatively, a TLS may have the sequence of a precursor micro RNA or messenger RNA stem-loop structure mediating intercellular RNA transport (Takeda et al., Plant Cell. 2011 Jan, 23(1): 258-272; Bussiere et al., J Virol, 2000 Mar, 74(6):2647-54).

The term "hybridization" as used herein refers to a single stranded DNA or RNA sequence with a specific sequence annealing to a complement DNA or RNA sequence. Single stranded DNA can also hybridize with single stranded RNA to result in a DNA/RNA hybrid. Usually, a double-stranded DNA or RNA or a hybrid is stable under physiological conditions. An increase in temperature will usually cause the two hybridized or annealed strands to separate into single strands. A decrease in temperature causes the single stranded DNA and/or RNA molecules to anneal or hybridize to each other. Hybridization involves the formation of base pairs between A and T (or U) nucleotides and G and C nucleotides of the specific sequence and the complement sequence. "Hybridization" is usually carried out under stringent conditions, preferably under high stringency conditions. The term "high stringency conditions" is to be understood such that a specific sequence specifically hybridizes to a complement sequence in an amount that is detectably stronger than non-specific hybridization. High stringency conditions include conditions which distinguish an oligonucleotide with an exact complement sequence, or an oligonucleotide containing only a few mismatched nucleotides (e.g. 1, 2, 3, 4 or 5 mismatched nucleotides), from a random sequence that happens to have a few small complement regions (comprised of e.g. 3 to 4 nucleotides) to the specific sequence. Such small regions of complementarity melt more easily than a longer complement sequence of preferably about 10 to about 25 nucleotides, and high stringency hybridization makes them easily distinguishable. Relatively high stringency conditions include, for example, low salt and/or high temperature conditions, such as provided by about 0.02-0.1 M NaCl or the equivalent, at temperatures of about 50°C to about 70° C. Such high stringency conditions tolerate little, if any, mismatch between a specific sequence and a complement sequence. It is generally appreciated that conditions can be rendered more stringent by the addition of increasing amounts of formamide.

The term "complementary" means that a specific predetermined nucleotide sequence is either completely (which may be preferred) or in most parts the complement sequence of an underlying nucleotide sequence. Thus, put in other words, a complementary sequence is either 100% identical (which may be preferred) or is identical to a high degree to the complement sequence of the underlying sequence. When a nucleotide sequence is referred to as complementary, it is meant that it is complementary to such a degree that hybridization will take place specifically between it and its complement sequence. Accordingly, the complementary sequence is complementary to its complement sequence to such a degree that no hybridization between it and a non-complementary sequence takes place. It is generally preferred that the complement sequence of the oligonucleotide is 100% identical to the complement sequence of the underlying target sequence. However, in some embodiments, complementarity may be 80%, 81%, 82%, 83%, 84%, 85%, 86%, 87%, 88%, 89%, 90%, 91%, 92%, 93%, 94%, 95%, 96%, 97%, 98%, or 99% across the entire length of the complementary sequences. When the complementarity is below 100%, it is preferred that the complementary sequences include regions of at least 50 nucleotides in length which are 100% complementary.

The term "sequence identity" as used herein means that two nucleotide sequences are identical if they exhibit the same length and order of nucleotides. The percentage of identity typically describes the extent to which two sequences are identical, i.e. it typically describes the percentage of nucleotides that correspond in their sequence position to identical nucleotides of a reference sequence. For the determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the complete first sequence. In other words, in the context of the present invention, identity of sequences preferably relates to the percentage of nucleotides of a sequence, which have the same position in two sequences having the same length.

A "pharmaceutical composition" is a composition that is suitable for administration to a subject (e.g. a plant or an animal), and comprises a pharmaceutically acceptable carrier. The term "pharmaceutically acceptable carrier" as used herein as used herein is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the circular RNA of the invention, use thereof in the compositions is contemplated.

Pharmaceutical compositions may further comprise a permeability-enhancing agent, an adjuvant, a peptide-carrier, a vesicular endocytosis-carrier, a micro-carrier, and/or a nano-carrier. A "permeability-enhancing agent" is any agent which facilitates the uptake of the composition, especially the circular RNA of the invention, into a cell or tissue of an organism and may, e.g., be selected from the group consisting of a surfactant, and organic solvent, an aqueous solution of an organic solvent, an oxidizing agent, an acid, a base, an oil, an enzyme, or a combination thereof. An "adjuvant" stimulates the immune response of the subject the pharmaceutical composition is administered to, any may or may not itself be immunogenic. Adjuvants include, e.g., analgesic adjuvants, inorganic compounds such as alum, aluminium hydroxide, aluminium phosphate, calcium phosphate hydroxide, mineral oils such as paraffin oil, bacterial products such as killed bacteria *(Bordetella pertussis, Mycobacterium bovis*) and toxoids, nonbacterial organics such as squalene, plant saponins from Quillaja (See Quillaia), soybean, Polygala senega, cytokines such as IL-1, IL-2, IL-12, combination adjuvants such as Freund's complete adjuvant and Freund's incomplete adjuvant, and food-based oils. "Peptide carriers" include, e.g., cell-penetrating peptides (CPPs), such as hydrophilic cationic peptides and amphiphilic CPPs, endosome disrupting peptides, and multifunctional peptides, and are described, e.g., in Tai and Gao (2016), Adv Drug Deliv Rev 110-111: p.157-168.

The term "synthesizing" as used herein refers to the in vitro production of RNA molecules. The first step in in vitro RNA synthesis is to prepare the DNA template (e.g. cDNA) corresponding to the RNA sequence to be synthesized. Linearized plasmid DNA, PCR products, and synthetic oligonucleotides can be used as templates for transcription reactions. The template DNA is transcribed by a T7, T3 or SP6 RNA phage or other polymerase with similar RNA synthesis function in the presence of ribonucleoside triphosphates (rNTPs). The polymerase traverses the template strand and uses base pairing with the DNA to synthesize a complementary RNA strand (using uracil in the place of thymine). The RNA polymerase travels from the 3' → 5' end of the DNA template strand, to produce an RNA molecule in the 5' → 3' direction. After successful in vitro transcription, the template DNA is degraded with a DNAse such as DNasel. Synthesized RNAs can also be affinity purified before further use.

The term "ligating" as used herein refers to the joining of a 5' single stranded RNA end to a 3' single stranded RNA end. For example, T4 RNA Ligase 1 catalyzes the ligation of a 5' phosphoryl-terminated nucleic acid donor to a 3' hydroxyl-terminated nucleic acid acceptor through the formation of a 3' → 5' phosphodiester bond with hydrolysis of ATP to AMP and PPi.

A "methylation motif" as used herein means a sequence motif within an RNA that is recognized by RNA methyltransferases such as RNA:m5C methyltransferases (RCMTs), which then convert cytosine within the RNA into 5-methylcytosine (m5C). A comprehensive overview of the role of m5C and RCMTs as well as the required motifs is provided in Cheng et al. (2018), Nature Communications 9: 1163.

An "endogenous" gene or transcript as used herein means a gene or transcript naturally found within the cell that is contacted with the circular RNAs or compositions of the invention. A "transgene" is a gene not naturally found within the cell that is contacted with the circular RNAs or compositions of the invention, i.e. which was introduced into the genome of the cell at an ectopic site by means of genetic engineering.

"Soaking" refers to submerging an organism or part of an organism (typically a plant) in a solution of the circular RNA or composition of the invention for an extended period of time.

"Coating" refers to covering an organism or part of an organism with the circular RNA or composition of the invention or a solution thereof for an extended period of time.

"Spraying" refers to the application of droplets of a solution comprising the circular RNA or composition of the invention to an organism or part of an organism.

"Biolistically introducing" refers to delivery of nucleic acid to cells by high-speed particle bombardment. The technique uses circular-RNA-coated particles propelled by a pressurized gun (gene gun) directed at an organism or part of an organism to transfect the organism or part of the organism.

"Electroporating" refers to a technique in which an electrical field is applied to cells in order to increase the permeability of the cell membrane, allowing the circular RNA or composition of the invention to be introduced into the cell (also called electrotransfer).

"High-pressure spraying" refers to using a conventional compressor and e.g. an air brush pistol to spray a solution of the circular RNA or composition of the invention at high velocity, and is described, e.g., in Dalakouras et al. (2016), Front Plant Sci 7, 1327.

"Callus tissue" of plants is a growing mass of unorganized plant parenchyma cells. In living plants, callus cells are those cells that cover a plant wound. Callus tissue forms, e.g., after cutting a plant for grafting purposes, i.e. when a first plant is cut and a cut part of a second plant is applied to the cut site, thereby joining the plant tissues to continue their growth jointly. The upper part of the combined plant is called the scion while the lower part is called the rootstock. The success of this joining requires that the vascular tissues of the two plant parts grow together, which joining is called inosculation.

"Suppression" of a pathogen refers to inhibiting or entirely preventing the growth and expansion of pathogens, e.g. by suspending growth of the pathogen or by killing the pathogen.

The expression of a gene is "dysregulated" in a cell when the cell or a comparable cell in normal, healthy conditions, expresses the gene at lower levels, and the altered (dysregulated) expression within the cell leads to an unhealthy or abnormal state. Similarly, the function of a transcript is "dysregulated" in a cell when the cell or a comparable cell in normal, healthy conditions, exhibits the function of the transcript at lower levels, and the altered (dysregulated) function within the cell leads to an unhealthy or abnormal state. Accordingly, "better regulation" refers to the inhibition of the expression of the gene or function of the transcript such that the resulting level of expression/function is lowered. The lowered expression/function more closely resembles the expression/function in a normal, healthy state of the cell. A "disease involving the dysregulation of the expression of a gene" thus is the unhealthy state resulting in an organism all or some cells of which exhibit dysregulation of the expression of a gene. A "disease involving the dysregulation of the function of a transcript" similarly is the unhealthy state resulting in an organism all or some cells of which exhibit dysregulation of the function of a transcript.

"Infectious disease" refers to disorders caused by organisms infecting another organism. The infectious organism may be, e.g., a pathogenic bacterium, virus, fungus, or parasite.

A "feeding animal" is an animal that feeds off an organism of interest. Examples include, but are not limited to, carnivorous and herbivorous invertebrates and vertebrates such as insects, birds, mammals, or reptiles.

A "heritable disease" is a genetic order, i.e. a disorder caused by one or more mutations in the genome of an organism that can be passed on to future generations. Examples include, but are not limited to, single gene disorders or multifactorial disorders such as Polycystic kidney disease, Cystic fibrosis, diabetes, heart diseases, or Duchenne muscular dystrophy.

### Detailed Description of the Invention

The invention will be described in more detail in the following.

### First aspect: circular RNA

In an embodiment of a first aspect, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other. A schematic depiction of a single-chain circular RNA is depicted in Figure 1C.

In an embodiment, either the sense strand sequence or the antisense strand sequence contains a nick.

In a second embodiment of the first aspect, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other. A schematic depiction of a circular RNA is depicted in Figure 1B (top).

In an embodiment, in any of the above embodiments, the second loop sequence or the loop sequence of the second single-chain RNA molecule is a stem-loop sequence and comprises a tRNA-like sequence (TLS) motif structure. Schematic depiction of circular RNAs comprising RLS motifs are depicted in Figure 1B (second from top, second from bottom, and bottom).

That is, in an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different or the same sequences, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

In an embodiment, the first loop sequence orthe loop sequence is also a stem-loop sequence and comprises a tRNA-like sequence (TLS) motif structure.

That is, in an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

In an embodiment, the TLS motif structure is
a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
c. a viral 3' TLS sequences from a virus forming a tRNA related structure.
d. a viroid forming stem-loop or pseudo-knot structures related to tRNAs, or
e. a precursor micro RNA or messenger RNA stem-loop structure mediating intercellular RNA transport.

That is, in an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the TLS motif structure is
a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
c. a viral 3' TLS sequences from a virus forming a tRNA related structure,
d. a viroid forming stem-loop or pseudo-knot structures related to tRNAs, or
e. a precursor micro RNA or messenger RNA stem-loop structure mediating intercellular RNA transport;
wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d.a viroid forming stem-loop or pseudo-knot structures related to tRNAs, or
      e. a precursor micro RNA or messenger RNA stem-loop structure mediating intercellular RNA transport; and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different or the same sequences, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

That is, in an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport;
      and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   v. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport;
      and
   iii. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

In an embodiment, the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues.

That is, in an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues..

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues..

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues.. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues..

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the TLS motif structure is
a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
c. a viral 3' TLS sequences from a virus forming a tRNA related structure,
d. a viroid forming stem-loop or pseudo-knot structures related to tRNAs, or
e. a precursor micro RNA or messenger RNA stem-loop structure mediating intercellular RNA transport;
wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues.. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      a.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport; and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues..

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other, wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues.. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport;
      and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport;
      and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues.

In an embodiment, in any of the above embodiments, the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length.

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length..

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length.

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different or the same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length.

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the TLS motif structure is
a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
c. a viral 3' TLS sequences from a virus forming a tRNA related structure,
d. a viroid forming stem-loop or pseudo-knot structures related to tRNAs, or
e. a precursor micro RNA or messenger RNA stem-loop structure mediating intercellular RNA transport;
wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport; and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length.

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport;
      and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport;
      and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length.

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues..

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues. In a preferred embodiment, all cytosine residues of the circular RNA are m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different or the same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues..

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, and wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different or the same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues.

In an embodiment, the present invention provides a single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the second loop sequence is a stem-loop sequence and comprises a tRNA-like sequence (TLS) motif structure, wherein the TLS motif structure is
a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
c. a viral 3' TLS sequences from a virus forming a tRNA related structure,
d. a viroid forming stem-loop or pseudo-knot structures related to tRNAs, or
e. a precursor micro RNA or messenger RNA stem-loop structure mediating intercellular RNA transport;
wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, wherein the circular RNA of any of the above embodiments comprises at least one methylated cytosine (m⁵C) residue, e.g. 1,2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport; and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different or the same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues..

In an embodiment, the present invention provides a single-chain circular RNA comprising:
a. a sense strand sequence,
b. an antisense strand sequence complementary to the sense strand sequence,
   c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the first and second loop sequences are a stem-loop sequences and comprise a tRNA-like sequence (TLS) motif structure, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other, wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues. In an embodiment, either the sense strand sequence or the antisense strand sequence contains a nick.

In an embodiment, the present invention provides a circular RNA consisting of:
a. a first single-chain RNA molecule consisting of:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, is at least 3 nucleotides in length, is a stem-loop sequence and comprises a tRNA-like sequence (TLS) motif structure, wherein the TLS motif structure is
      a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      b.selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      c. a viral 3' TLS sequences from a virus forming a tRNA related structure,
      d. a viroid forming stem-loop or pseudo-knot structures related to tRNAs, or
      e. a precursor micro RNA or messenger RNA stem-loop structure mediating intercellular RNA transport;
      and
   iv. a second pairing sequence located 3' or 5' of the loop sequence, wherein the second pairing sequence is complementary to the first pairing sequence,
   wherein the first and second pairing sequences are paired to form a stem; and
b. a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, is a stem-loop sequence and comprises a tRNA-like sequence (TLS) motif structure, wherein the TLS motif structure is
      f. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
      g. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
      h. a viral 3' TLS sequences from a virus forming a tRNA related structure,
      i. a viroid forming stem-loop or pseudo-knot structures related to tRNAs, or
      j. a precursor micro RNA or messenger RNA stem-loop structure mediating intercellular RNA transport;
      and
   iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different or the same sequences, wherein the sense strand sequence and the antisense strand sequence are paired to form a stem, wherein the sense and antisense strand sequences correspond to nucleotide sequences of a target gene or target transcript, wherein the nucleotide sequences of the target gene or target transcript are complementary to each other, wherein the circular RNA of any of the above embodiments comprises at least one methylated cytosine (m⁵C) residue, e.g. 1, 2, 3, 4, 5, 6, 7, 8, 9, 10, 11, 12, 13, 14, 15, 16, 17, 18, 19, 20, or more m⁵C residues, and wherein the stem formed from the sense strand sequence and the antisense strand sequence is 19 base pairs or longer in length. In a preferred embodiment, the stem is 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 base pairs or longer in length. In a preferred embodiment, all cytosine residues of the circular RNA are m⁵C residues.

### Second aspect: composition comprising the circular RNA of the first aspect

In a second aspect, the present invention provides a composition comprising the circular RNA of any one of the embodiments of the first aspect.

In an embodiment, the composition is a pharmaceutical composition further comprising a pharmaceutically acceptable carrier.

In an embodiment, the composition of any one of the above embodiments further comprises
a. a permeability-enhancing agent, optionally wherein the permeability-enhancing agent is selected from the group consisting of a surfactant, and organic solvent, an aqueous solution of an organic solvent, an oxidizing agent, an acid, a base, an oil, an enzyme, or a combination thereof; or
b. an adjuvant; or
c. a peptide-carrier; or
d. a vesicular endocytosis-carrier; or
e. a micro-carrier; or
f. a nano-carrier.

That is, in an embodiment, the present invention provides a composition comprising the circular RNA of any one of the embodiments of the first aspect and
a. a permeability-enhancing agent, optionally wherein the permeability-enhancing agent is selected from the group consisting of a surfactant, and organic solvent, an aqueous solution of an organic solvent, an oxidizing agent, an acid, a base, an oil, an enzyme, or a combination thereof; or
b. an adjuvant; or
c. a peptide-carrier; or
d. a vesicular endocytosis-carrier; or
e. a micro-carrier; or
f. a nano-carrier.

In an embodiment, the present invention provides a pharmaceutical composition comprising the circular RNA of any one of the embodiments of the first aspect, a pharmaceutically acceptable carrier, and
a. a permeability-enhancing agent, optionally wherein the permeability-enhancing agent is selected from the group consisting of a surfactant, and organic solvent, an aqueous solution of an organic solvent, an oxidizing agent, an acid, a base, an oil, an enzyme, or a combination thereof; or
b. an adjuvant; or
c. a peptide-carrier; or
d. a vesicular endocytosis-carrier; or
e. a micro-carrier; or
f. a nano-carrier.

### Third aspect: kit comprising the circular RNA of the first aspect or the compositions of the second aspect

In a third aspect, the present invention provides a kit comprising the circular RNA of any one of the embodiments of the first aspect or the composition of any one of the embodiments of the second aspect.

### Fourth aspect: method of producing the circular RNA of the first aspect

In an embodiment of a fourth aspect, the present invention provides a method of producing a circular RNA, comprising the steps of:
a. synthesizing a first single-chain RNA molecule comprising:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, optionally wherein the loop sequence is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem;
b. synthesizing a second single-chain RNA molecule comprising
   v. an antisense strand sequence complementary to the sense strand sequence of the first single-chain RNA molecule,
   vi. a first pairing sequence located 3' or 5' of the antisense strand sequence,
   vii. a loop sequence located 3' or 5' of the antisense strand sequence, wherein the loop sequence is at least 3 nucleotides in length, optionally wherein the loop sequence is a stem-loop sequence and comprises a tRNA-like sequence (TLS) motif structure, and
   viii. a second pairing sequence located 3' or 5' of the loop sequence, wherein the second pairing sequence is complementary to the first pairing sequence,
   wherein the first and second pairing sequences are paired to form a stem;
c. hybridizing the first and second single-chain RNA molecules such that the sense and antisense strand sequences pair to form a stem, thereby producing a double-chain circular RNA; and
d. optionally ligating the hybridized first and second single-chain RNA molecules of step c to form a single-chain circular RNA,
wherein the locations in the first and second single-chain RNA molecule are all 3' or all 5', wherein the loop sequence of the first and the loop sequence of the second single-chain RNA molecules have different or the same sequences, wherein the sense and antisense strand sequences correspond to nucleotide sequences of a target gene or target transcript, and wherein the nucleotide sequences of the target gene and or transcript are complementary to each other. A schematic depiction of the first and second single-chain RNA molecules of steps a and b are depicted in Figure 1A (top), and a schematic depiction of the double-chain circular RNA resulting from step c is depicted in Figure 1A (bottom). A schematic depiction of the single chain circular RNA molecule resulting from step d is depicted in Figure 1C.

In another embodiment of the fourth aspect, the present invention provides a method of producing a circular RNA, comprising the steps of:
a. synthesizing a single-chain RNA molecule comprising:
   i. a sense strand sequence,
   ii. a first pairing sequence located 3' or 5' of the sense strand sequence,
   iii. a first loop sequence located 3' or 5' of the first pairing sequence, wherein the first loop sequence is at least 3 nucleotides in length, and
   iv. a second pairing sequence located 3' or 5' of the first loop sequence, wherein the second pairing sequence is complementary to the first pairing sequence,
   v. an antisense strand sequence complementary to the sense strand sequence, located 3' or 5' of the second pairing sequence; and
   vi. a loop sequence located 3' or 5' of the antisense strand sequence, optionally wherein the second loop sequence comprises a tRNA-like sequence (TLS) motif structure, and
   wherein the locations are all 3' or all 5',
b. hybridizing the single-chain RNA molecule such that the sense and antisense strand sequences pair to form a stem and the first and second pairing sequences from a stem, thereby producing a circular single-chain RNA; and
c. optionally ligating the hybridized circular single-chain RNA molecules of step b,
wherein the first and second loop sequences have different or the same sequences, wherein the sense and antisense strand sequences correspond to nucleotide sequences of a target gene or target transcript, and wherein the nucleotide sequences of the target gene and or transcript are complementary to each other. A schematic depiction of the single chain circular RNA molecule resulting from step c is depicted in Figure 1C.

In an embodiment, in anyone of the above methods, in steps a and b 5-methylcytidine-5'triphosphate is provided.

That is, in an embodiment, the present invention provides a method of producing a circular RNA, comprising the steps of:
a. synthesizing a first single-chain RNA molecule comprising:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, optionally wherein the loop sequence is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem;
b. synthesizing a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, optionally wherein the loop sequence is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem;
c. hybridizing the first and second single-chain RNA molecules such that the sense and antisense strand sequences pair to form a stem, thereby producing a double-chain circular RNA; and
d. optionally ligating the hybridized first and second single-chain RNA molecules of step c to form a single-chain circular RNA,
wherein in steps a and b 5-methylcytidine-5'triphosphate is provided, wherein the locations in the first and second single-chain RNA molecule are all 3' or all 5', wherein the loop sequence of the first and the loop sequence of the second single-chain RNA molecules have different or the same sequences, wherein the sense and antisense strand sequences correspond to nucleotide sequences of a target gene or target transcript, and wherein the nucleotide sequences of the target gene and or transcript are complementary to each other.

In another embodiment of the fourth aspect, the present invention provides a method of producing a circular RNA, comprising the steps of:
a. synthesizing a single-chain RNA molecule comprising:
   i. a sense strand sequence,
   ii. a first pairing sequence located 3' or 5' of the sense strand sequence,
   iii. a first loop sequence located 3' or 5' of the first pairing sequence, wherein the first loop sequence is at least 3 nucleotides in length, and
   iv. a second pairing sequence located 3' or 5' of the first loop sequence, wherein the second pairing sequence is complementary to the first pairing sequence,
   v. an antisense strand sequence complementary to the sense strand sequence, located 3' or 5' of the second pairing sequence; and
   vi. a loop sequence located 3' or 5' of the antisense strand sequence, optionally wherein the second loop sequence comprises a tRNA-like sequence (TLS) motif structure, and
   wherein the locations are all 3' or all 5',
b. hybridizing the single-chain RNA molecule such that the sense and antisense strand sequences pair to form a stem and the first and second pairing sequences from a stem, thereby producing a circular single-chain RNA; and
c. optionally ligating the hybridized circular single-chain RNA molecules of step b,
wherein in steps a and b 5-methylcytidine-5'triphosphate is provided, wherein the first and second loop sequences have different or the same sequences, wherein the sense and antisense strand sequences correspond to nucleotide sequences of a target gene or target transcript, and wherein the nucleotide sequences of the target gene and or transcript are complementary to each other.

In another embodiment, the circular RNA comprises a methylation motif and the method further comprises exposing the circular RNA to a methyltransferase enzyme modifying cytosine (C) to 5-methylcytosine (m5C) RNA in vivo, ex vivo, or in vitro.

That is, in an embodiment, the present invention provides a method of producing a circular RNA, comprising the steps of:
a. synthesizing a first single-chain RNA molecule comprising:
   i. a sense strand sequence,
   ii. a first pairing sequence located 3' or 5' of the sense strand sequence,
   iii. a loop sequence located 3' or 5' of the first pairing sequence, wherein the loop sequence is at least 3 nucleotides in length, optionally wherein the loop sequence is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem;
b. synthesizing a second single-chain RNA molecule comprising
   i. an antisense strand sequence complementary to the sense strand sequence of the first single-chain RNA molecule,
   ii. a first pairing sequence located 3' or 5' of the antisense strand sequence,
   iii. a loop sequence located 3' or 5' of the antisense strand sequence, wherein the loop sequence is at least 3 nucleotides in length, optionally wherein the loop sequence is a stem-loop sequence and comprises a tRNA-like sequence (TLS) motif structure, and
   iv. a second pairing sequence located 3' or 5' of the loop sequence, wherein the second pairing sequence is complementary to the first pairing sequence,
   wherein the first and second pairing sequences are paired to form a stem;
c. hybridizing the first and second single-chain RNA molecules such that the sense and antisense strand sequences pair to form a stem, thereby producing a double-chain circular RNA; and
d. optionally ligating the hybridized first and second single-chain RNA molecules of step c to form a single-chain circular RNA,
wherein the locations in the first and second single-chain RNA molecule are all 3' or all 5', wherein the loop sequence of the first and the loop sequence of the second single-chain RNA molecules have different or the same sequences, wherein the sense and antisense strand sequences correspond to nucleotide sequences of a target gene or target transcript, wherein the nucleotide sequences of the target gene and or transcript are complementary to each other, and wherein the circular RNA comprises a methylation motif and the method further comprises exposing the circular RNA to a methyltransferase enzyme modifying cytosine (C) to 5-methylcytosine (m5C) RNA in vivo, ex vivo, or in vitro.

In another embodiment of the fourth aspect, the present invention provides a method of producing a circular RNA, comprising the steps of:
d. synthesizing a single-chain RNA molecule comprising:
   vii. a sense strand sequence,
   viii. a first pairing sequence located 3' or 5' of the sense strand sequence,
   ix. a first loop sequence located 3' or 5' of the first pairing sequence, wherein the first loop sequence is at least 3 nucleotides in length, and
   x. a second pairing sequence located 3' or 5' of the first loop sequence, wherein the second pairing sequence is complementary to the first pairing sequence,
   xi. an antisense strand sequence complementary to the sense strand sequence, located 3' or 5' of the second pairing sequence; and
   xii. a loop sequence located 3' or 5' of the antisense strand sequence, optionally wherein the second loop sequence comprises a tRNA-like sequence (TLS) motif structure, and
   wherein the locations are all 3' or all 5',
e. hybridizing the single-chain RNA molecule such that the sense and antisense strand sequences pair to form a stem and the first and second pairing sequences from a stem, thereby producing a circular single-chain RNA; and
f. optionally ligating the hybridized circular single-chain RNA molecules of step b,
wherein the first and second loop sequences have different or the same sequences, wherein the sense and antisense strand sequences correspond to nucleotide sequences of a target gene or target transcript, wherein the nucleotide sequences of the target gene and or transcript are complementary to each other, and wherein the circular RNA comprises a methylation motif and the method further comprises exposing the circular RNA to a methyltransferase enzyme modifying cytosine (C) to 5-methylcytosine (m5C) RNA in vivo, ex vivo, or in vitro.

### Fifth aspect: method of inhibiting the expression of a target gene or the function of a target transcript in a cell

In a fifth aspect, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other.

In an embodiment, the method is performed *in vitro, ex vivo,* or *in vivo.*

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other.

In an embodiment of any of the above methods of the fifth aspect, the cell is a cell selected from the group consisting of a eukaryotic cell, optionally wherein the cell is an animal cell, a fungal cell, or a plant cell.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein the cell is a cell selected from the group consisting of a eukaryotic cell, optionally wherein the cell is an animal cell, a fungal cell, or a plant cell.

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein the cell is a cell selected from the group consisting of a eukaryotic cell, optionally wherein the cell is an animal cell, a fungal cell, or a plant cell.

In an embodiment of any of the above methods of the fifth aspect, the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant.

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, the cell is a cell selected from the group consisting of a eukaryotic cell, optionally wherein the cell is an animal cell, a fungal cell, or a plant cell, and wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant.

In an embodiment of any of the above methods of the fifth aspect, the cell is present in an organism, and the circular RNA or composition is introduced within a body cavity of the organism inside or outside the cell.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein he cell is present in an organism, and the circular RNA or composition is introduced within a body cavity of the organism inside or outside the cell.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and the circular RNA or composition is introduced within a body cavity of the organism inside or outside the cell.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced within a body cavity of the organism inside or outside the cell.

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced within a body cavity of the organism inside or outside the cell.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced within a body cavity of the organism inside or outside the cell.

In an embodiment, the present invention provides an *in vitro, ex vivo, or in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced within a body cavity of the organism inside or outside the cell.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced within a body cavity of the organism inside or outside the cell.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced within a body cavity of the organism inside or outside the cell.

In an embodiment another embodiment of the above methods of the fifth aspect the cell is present in an organism, and the circular RNA or composition is introduced into the organism by extracellular injection.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein he cell is present in an organism, and the circular RNA or composition is introduced into the organism by extracellular injection.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and the circular RNA or composition is introduced into the organism by extracellular injection.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced into the organism by extracellular injection.

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced into the organism by extracellular injection.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by extracellular injection.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by extracellular injection.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by extracellular injection.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by extracellular injection.

In another embodiment, the cell is present in an organism, and the circular RNA or composition is introduced into the organism by feeding.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein he cell is present in an organism, and the circular RNA or composition is introduced into the organism by feeding.

In an embodiment, the present invention provides an *in vitro, ex vivo, or in vivo* method of inhibiting the expression of a target gene or the function of a target transcript in a cell comprising contacting the cell with the circular RNA of any one of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and the circular RNA or composition is introduced into the organism by feeding.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced into the organism by feeding.

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced into the organism by feeding.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by feeding.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by feeding.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by feeding.

In an embodiment, the present invention provides an *in vitro, ex vivo, or in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the organism by feeding.

In yet another embodiment, the cell is a plant cell present in a plant, and the circular RNA or composition is topically applied to the surface of the plant, optionally wherein the circular RNA or composition is applied by soaking, coating, spraying, biolistically introducing, electroporating or high-pressure spraying.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein the cell is present in an organism, and the circular RNA or composition is topically applied to the surface of the plant, optionally wherein the circular RNA or composition is applied by soaking, coating, spraying, biolistically introducing, electroporating or high-pressure spraying.

In an embodiment, the present invention provides an *in vitro, ex vivo, or in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and the circular RNA or composition is topically applied to the surface of the plant, optionally wherein the circular RNA or composition is applied by soaking, coating, spraying, biolistically introducing, electroporating or high-pressure spraying.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is topically applied to the surface of the plant, optionally wherein the circular RNA or composition is applied by soaking, coating, spraying, biolistically introducing, electroporating or high-pressure spraying.

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is topically applied to the surface of the plant, optionally wherein the circular RNA or composition is applied by soaking, coating, spraying, biolistically introducing, electroporating or high-pressure spraying.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is topically applied to the surface of the plant, optionally wherein the circular RNA or composition is applied by soaking, coating, spraying, biolistically introducing, electroporating or high-pressure spraying.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is topically applied to the surface of the plant, optionally wherein the circular RNA or composition is applied by soaking, coating, spraying, biolistically introducing, electroporating or high-pressure spraying.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is topically applied to the surface of the plant, optionally wherein the circular RNA or composition is applied by soaking, coating, spraying, biolistically introducing, electroporating or high-pressure spraying.

In an embodiment, the present invention provides an *in vitro, ex vivo, or in vivo* method of inhibiting the expression of a target gene or the function of a target transcript in a cell comprising contacting the cell with the circular RNA of anyone of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is topically applied to the surface of the plant, optionally wherein the circular RNA or composition is applied by soaking, coating, spraying, biolistically introducing, electroporating or high-pressure spraying.

In yet another embodiment, the cell is a plant cell present in a plant, and the circular RNA or composition is introduced into the plant by applying the circular RNA or composition on plant tissue after cutting the plant or on callus tissue formed after cutting the plant, optionally wherein the plant is a grape vine.

That is, in an embodiment, the present invention provides a method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and wherein the cell is present in an organism, and the circular RNA or composition is introduced into the plant by applying the circular RNA or composition on plant tissue after cutting the plant or on callus tissue formed after cutting the plant, optionally wherein the plant is a grape vine.

In an embodiment, the present invention provides an *in vitro, ex vivo, or in vivo* method of inhibiting the expression of a target gene or the function of a target transcript in a cell comprising contacting the cell with the circular RNA of any one of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, and the circular RNA or composition is introduced into the plant by applying the circular RNA or composition on plant tissue after cutting the plant or on callus tissue formed after cutting the plant, optionally wherein the plant is a grape vine.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced into the plant by applying the circular RNA or composition on plant tissue after cutting the plant or on callus tissue formed after cutting the plant, optionally wherein the plant is a grape vine.

That is, the present invention provides an *in vitro, ex vivo,* or *in vivo* method of inhibiting the expression of a target gene or the function of a target transcript in a cell comprising contacting the cell with the circular RNA of any one of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, and the circular RNA or composition is introduced into the plant by applying the circular RNA or composition on plant tissue after cutting the plant or on callus tissue formed after cutting the plant, optionally wherein the plant is a grape vine.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the plant by applying the circular RNA or composition on plant tissue after cutting the plant or on callus tissue formed after cutting the plant, optionally wherein the plant is a grape vine.

In an embodiment, the present invention provides an *in vitro, ex vivo, or in vivo* method of inhibiting the expression of a target gene or the function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the plant by applying the circular RNA or composition on plant tissue after cutting the plant or on callus tissue formed after cutting the plant, optionally wherein the plant is a grape vine.

In an embodiment, the present invention provides a method of inhibiting the expression of a target gene orthe function of a target transcript in a cell comprising contacting the cell with the circular RNA of any one of the embodiments of the first aspect or the composition of any one of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, wherein the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the plant by applying the circular RNA or composition on plant tissue after cutting the plant or on callus tissue formed after cutting the plant, optionally wherein the plant is a grape vine.

In an embodiment, the present invention provides an *in vitro, ex vivo, or in vivo* method of inhibiting the expression of a target gene or the function of a target transcript in a cell comprising contacting the cell with the circular RNA of any one of the embodiments of the first aspect or the composition of anyone of the embodiments of the second aspect in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other, the cell is a cell selected from the group consisting of a eukaryotic cell, optionally wherein the cell is an animal cell, a fungal cell, or a plant cell, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant, and the circular RNA or composition is introduced into the plant by applying the circular RNA or composition on plant tissue after cutting the plant or on callus tissue formed after cutting the plant, optionally wherein the plant is a grape vine.

In an embodiment of any of the above methods of the fifth aspect, the expression of the target gene or function of the target transcript is inhibited by at least 10%, 15%, 20%, 25%, 30%, 35%, 40%, 45%, 50%, 55%, 60%, 65%, 70%, 75%, 80%, 85%, 90%, 95%, 96%, 97%, 98%, or 99%.

In an embodiment of any of the above methods of the fifth aspect, inhibiting the expression of a target gene or the function of a target transcript results in suppression of a pathogen.

That is, in an embodiment of any of the above methods of the fifth aspect, the expression of the target gene or function of the target transcript is inhibited by at least 10%, 15%, 20%, 25%, 30%, 35%, 40%, 45%, 50%, 55%, 60%, 65%, 70%, 75%, 80%, 85%, 90%, 95%, 96%, 97%, 98%, or 99%, and inhibiting the expression of a target gene or the function of a target transcript results in suppression of a pathogen.

In another embodiment, the expression of the target gene or the function of the target transcript is dysregulated in the cell and inhibiting the expression of a target gene or function of the target transcript results in better regulation.

That is, in an embodiment of any of the above methods of the fifth aspect, the expression of the target gene or function of the target transcript is inhibited by at least 10%, 15%, 20%, 25%, 30%, 35%, 40%, 45%, 50%, 55%, 60%, 65%, 70%, 75%, 80%, 85%, 90%, 95%, 96%, 97%, 98%, or 99%, the expression of the target gene or the function of the target transcript is dysregulated in the cell, and inhibiting the expression of a target gene or function of the target transcript results in better regulation.

### Sixth aspect: circular RNA of the first aspect and composition of the second aspect for use

In a sixth aspect, the present invention provides the circular RNA of any one of the embodiments of the first aspect or the composition of any one of the embodiments of the second aspect for use in a method of treating a condition in a subject in need thereof, wherein the condition is selected from the group consisting of an infectious disease, feeding animals, a heritable disease, cancer, a disease involving the dysregulation of the expression of a gene, and a disease involving dysregulation of the function of a transcript.

### Examples

The following Examples are merely illustrative and shall describe the present invention in a further way. These Examples shall not be construed to limit the present invention thereto.

### Example 1; Methods and Materials

In the following Examples 2-5, the below methods and materials were used:

### Plant materials

*Arabidopsis thaliana* Col-0 (wild type), transgenic *35S::ER-GFP* (Haseloff et al., 1997, Proc Natl Acad Sci U S A 94, p.2122-2127), *35S::PEX11D-YFP* (Koch et al., 2010, J Cell Sci 123, p.3389-3400; Huber et al., 2012, Traffic 13, p.157-167), and dexamethasone (DEX) inducible *SHOOT-MERISTEMLESS (GR-STM)* (Gallois et al., 2002, Development 129, p.3207-3217; Lenhard et al., 2002, Development 129, p.3195-3206) *A. thaliana* Col-0 lines were used in this study as indicated. All seeds were sterilized by incubation in 1.2% Sodium hypochlorite with 0.01% Tween 20 for 3 minutes, followed by a 70% (v/v) ethanol wash for 2 to 3 minutes and 5 to 7 times washes with sterile water. Seeds were stratified for 1 to 2 days at 4 °C in dark. Seeds (5 to 6) were germinated in 96 well PCR plates containing 100 µL of ½ MS media (0.68% microagar, without sucrose) in a climate chamber under 12h light photoperiod (100 µmol m⁻² s⁻¹ photon flux density) at 21 °C/18°C (day/night) and 65% relative humidity. Five days after germination 10 µL of circRNAs (500 ng/µL) was added. After 2 to 3 days of RNA incubation the seedlings were transferred to square plates and grown further under the same conditions on 0.5 MS media (0.68% microagar, without sucrose) devoid of RNAs. *GR-STM* lines were induced by adding 10 µM DEX to the 0.5 MS medium in wells and plates.

### Single chain RNA molecule and circular RNA production

PCR primers with T7 promoter and YFP and STM matching sequences including HP, dDT, and/or TLS sequences were used to amplify cDNA templates (SEQ ID NOs: 1-8) as laid out in Table 1 using *A. thaliana* mRNA and reverse transcriptase. Note that the PCR cDNA template for PCR 4 was the PCR product 3 (matching sequence highlighted gray in Table 1).

First and second single chain RNA molecules were then generated from cDNA templates using T7 RNA polymerase as follows:
1. cDNA templates 1 & 2 (SEQ ID NOs: 1 and 2 forT7-YFP and SEQ ID NOs: 5 and 6 for T7-STM) were used to produce first and second single chain RNA molecules (SEQ ID NOs: 25 and 26 for T7-YFP and SEQ ID NOs: 29 and 30 for T7-STM), respectively, which were annealed to create nicked circular RNA (circRNA-2xHP) with two simple loops.
2. cDNA templates 2 & 3 (SEQ ID NOs: 2 and 3 for T7-YFP and SEQ ID NOs: 6 and 7 for T7-STM)) were used to produce second and first single chain RNA molecules (SEQ ID NOs: 26 and 27 for T7-YFP and SEQ ID NOs: 30 and 31 for T7-STM, respectively, which were annealed to create nicked circular RNA (circRNA-dDT) with one simple loop and one minimal TLS structure motif.
3. cDNA templates 2 & 4 (SEQ ID NOs: 2 and 4 for T7-YFP and SEQ ID NOs: 6 and 8 for T7-STM) were used to produce second and first single chain RNA molecules (SEQ ID NOs: 26 and 28 for T7-YFP and SEQ ID NOs: 30 and 31 for T7-STM, respectively, which were annealed to create nicked circular RNA (circRNA-TLS) with one simple loop and one full TLS structure motif.

Two separate T7 reactions were used to synthesize first and second single chain RNA molecules using a RiboMax Kit (RiboMAXTM Large Scale RNA Production System-T7 Cat# P1300, Promega). For *in vitro* synthesis of non-methylated first and second single chain RNA molecules, 20 mM of each rNTP was used. For the synthesis of methylated first and second single chain RNA molecules, rCTP nucleic bases were replaced with 20 mM m5CTP bases (5-Methylcytidine-5'triphosphate, Cat# N-1014, TriLink Biotechnologies). The quality and quantity of synthesized first and second single chain RNA molecules was estimated using both 1% Agarose gel electrophoresis and NanoDrop 2000. The first and second single chain RNA molecules were let to hybridize and form circular ("nicked") RNAs by heating to 80°C for 10 minutes and gradually cooled down to room temperature (1°C per minute) using a heat incubator for 1.5 mL tubes (Eppendorf ThermoMixer).

**Table 1. List of primers used to generate cDNA PCR products with T7 sequences to serve as templates for synthesizing first and second single chain RNA molecules for circRNAs.**

| **cDNA template created*** | **Forward PCR primer** | **Reverse PCR primer** |
|---|---|---|
| **T7-YFP-HP first single chain RNA molecule (1) (SEQ ID No: 1)** | TAA TAC GAC TCA CTA TA G ATG AAG GGC GAG GAG CTG TTC ACC GGG (SEQ ID NO: 9) | CGC CGG CCT CCG AAA CGG AGG CCG GCG G TTA CTT GTA CAG CTC GTC CAT GCC (SEQ ID NO: 10) |
| **T7-YFP-HP second single chain RNA molecule (2) (SEQ ID No: 2)** | CGC CGG CCT CCG AAA CGG AGG CCG GCG G ATG AAG GGC GAG GAG CTG TTC ACC GGG GGG (SEQ ID NO: 11) | TAA TAC GAC TCA CTA TA G TTA CTT GTA CAG CTC GTC CAT GCC GTG (SEQ ID NO: 12) |
| **T7-YFP-dDT first single chain RNA molecule (3) (SEQ ID No: 3)** | TAA TAC GAC TCA CTA TA G ATG AAG GGC GAG GAG CTG TTC ACC GGG (SEQ ID NO: 13) | TATCAGAGCGGACCTGTGGGTTAT GGGCCCACCACCA CTC TGA TA G TTA CTT GTA CAG CTC GTC CAT GCC GTG (SEQ ID NO: 14) |
| **T7-YFP-TLS first single chain RNA molecule (4) (SEQ ID No: 4)** | TAA TAC GAC TCA CTA TA G ATG AAG GGC GAG GAG CTG TTC ACC GGG (SEQ ID NO: 15) | TAT CAG AGC CAG GTT TCG ATC CTG GGA CCT GTG GGT T ATG GG CCC ACC ACG CTT CCG CTG CG CCA CTC TGA TA G TTA CTT GTA (SEQ ID NO: 16) |
| **T7-STM-HP first single chain RNA molecule (1) (SEQ ID No: 5)** | TAA TAC GAC TCA CTA TAG ATG GAG AGT GGT TCC AAC AGC (SEQ ID NO: 17) | CGC CGG CCT CCG AAA CGG AGG CCG GCG G TCA AAG CAT GGT GGA GGA GAT G (SEQ ID NO: 18) |
| **T7-STM-HP second single chain RNA molecule (2) (SEQ ID No: 6)** | CGC CGG CCT CCG AAA CGG AGG CCG GCG G ATG GAG AGT GGT TCC AAC AGC (SEQ ID NO: 19) | TAA TAC GAC TCA CTA TA G TCA AAG CAT GGT GGA GGA GAT G (SEQ ID NO: 20) |
| **T7-STM-dDT first single chain RNA molecule (3) (SEQ ID No: 7)** | TAA TAC GAC TCA CTA TA G ATG GAG AGT GGT TCC AAC AGC (SEQ ID NO: 21) | TATCAGAGCGGACCTGTGGGTTAT GGGCCCACCACCA CTC TGA TA G TCA AAG CAT GGT GGA GGA GAT G (SEQ ID NO: 22) |
| **T7-STM-TLS first single chain RNA molecule (4) (SEQ ID No: 8)** | TAA TAC GAC TCA CTA TA G ATG GAG AGT GGT TCC AAC AGC C (SEQ ID NO: 23) | TAT CAG AGC CAG GTT TCG ATC CTG GGA CCT GTG GGT T AT G GG CCC ACC ACG CTT CCG CTG CG CCA CTC TGA TA G TCA AAG CAT (SEQ ID NO: 24) |

T7 sequence present in PCR primers is shown in bold letters.

### Ligation of nicked circRNAs

To obtain full single chain circRNAs lacking nicks, *in vitro* synthesized annealed circRNAs were ligated. First, the circRNAs were dephosphorylated at 5'-end using Shrimp Alkaline Phosphatase (SAP, Product# 78390, Affymetrix USB) following the supplied protocol. The reaction was carried out in 20 µL containing 10 µg RNA, 2 µL 10X SAP Reaction Buffer (200 mM Tris-HCI pH 8.0, 100 mM MgCl2), 2.5 µL SAP (1 Unit/µL) at 37°C for 60 minutes and the reaction was stopped by heat inactivation at 65°C for 15 minutes. After ethanol precipitation the dephosphorylated RNA was phosphorylated by adding a single reactive phosphate moiety using T4 polynucleotide kinase (T4-PNK, Cat# M0201S, NEB Inc.) according the supplied protocol. In short, a 20 µL reaction mix containing 1x T4 PNK buffer (10 mM Tris-HCI, 50 mM KCI, 1 mM DTT, 0.1 mM EDTA, 50% Glycerol, 0.1 µM ATP, pH 7.4) and 1 Unit of T4-PNK was incubated for 30 minutes (37°C) followed by heating inactivation (65°C) for 20 minutes. After ethanol precipitation, the nicked ends of 4-5 µg nicked circRNA were ligated with 1 U of T4-RNA ligase with specific activity towards nicked dsRNA ends (Cat # M0239, NEB) in a 20 µL reaction mix for 30 minutes at 37°C followed by heat inactivation at 80°C for 5 minutes. Finally, the ligation reaction products were treated with the 3'-5' exoribonuclease Ribonuclease R (RNase R, Cat# RNR07250, Epicentre) to enrich ligated circRNAs.

In particular, annealed SEQ ID NOs: 25 and 26 for T7-YFP and SEQ ID NOs: 29 and 30 for T7-STM were ligated to create single-chain circular RNAs (circRNA-2xHP) with two simple loops (SEQ ID NO: 33 for T7-YFP and SEQ ID NO: 36 for T7-STM), annealed SEQ ID NOs: 25 and 27 for T7-YFP and SEQ ID NOs: 29 and 31 for T7-STM were ligated to create single-chain circular RNAs (circRNA-dDT) with one simple loop and one minimal TLS structure motif (SEQ ID NO: 34 for T7-YFP and SEQ ID NO: 37 for T7-STM), and annealed SEQ ID NOs: 25 and 28 for T7-YFP and SEQ ID NOs: 29 and 31 for T7-STM were ligated to create single-chain circular RNAs (circRNA-TLS) with one simple loop and one full TLS structure motif (SEQ ID NO: 35 for T7-YFP and SEQ ID NO: 38 for T7-STM).

### Melting curve analysis

To evaluate circRNAs and the effect of m⁵C modifications on stability, first and second single chain RNA molecules were synthesized using the T7 RNA synthesis protocol where all rATPs were replaced with 2-Aminopurine (2-AP, a fluorescent analogue of adenine base, Cat# NU-234S, Jena Bioscience). circRNAs without m⁵C modification and with m⁵C modification carrying 2-AP were obtained at a concentration of 100 ng/µL (in 0.1% DEPC treated sterile water) and used to acquire the spectra. The melting curve analysis was performed using Prometheus NanoTemper (NT.48, NanoTemper Technologies, GmbH), a nano-differential scanning fluorometry (nDSF) in 10 µL standard glass capillary. The ratio of fluorescent nucleotide (2-AP) emission at 330 and 350 nm was obtained and is used as an indicator of the shift of fluorescence emission upon unfolding. The melting curve was obtained by heating from 20°C to 95°C and emission spectra of unfolded RNA was recorded at every 2°C steps, simultaneously.

### Nuclease protection assays

Non-methylated and m5C sense *STM* ssRNA-HP (1 µg) was incubated with 0.01 U RNase A (RNase A, Cat. # R1253 Thermo Scientific^{™}) in 10mM Tris-HCI pH 7.6 for either 5 or 15 minutes at 37°C. For the nicked *STM* circRNA-TLS protection assay, 1 µg of annealed first and second single chain RNA molecules was digested with a mixture of 0.1 U RNase R and 0.001 U RNase A for 15 minutes at 37°C. Quality of ligated circRNA-dDT (1 µg) was evaluated by digesting with 0.1 U RNase R (RNase R, Cat. # RNR07250, Epicentre^{®}) for 15 minutes at 37°C using supplied RNase R buffer (0.2 M Tris-HCI pH 8.0, 1 M KCI, 1 mM MgCl₂). After completion of RNase treatment ∼50 ng of RNA samples were submitted to agarose gel-electrophoresis (1% Agarose, 1xTBE) for analysis.

### Confocal microscopy and fluorescence measurements

All ER-GFP and YFP-PEX11D plants were analyzed by CLSM (Leica TCS SP8, Leica Microsystems, Germany) for comparing fluorescence fusion protein presence and density changes due to silencing using the same gain (∼600 V) and pinhole (1 AU). Individual leaves of RNA treated and mock control plants were imaged in Z-stack mode using the following settings: samples were excited with Argon laser (65 mW, 20% output power). The ER-GFP fluorescence was excited with 488 nm laser and emission collected from 500 to 540 nm, YFP-PEX11D fluorescence was excited using 514 nm laser and emission was detected from 525 nm to 555 nm. The autofluorescence emission of plastids was simultaneously collected in the emission range from 690 to 750 nm. Z-stacks were collected in 15 steps at an interval of 10 -20 µm. Z-stack images were compiled and analyzed using the FIJI (ImageJ) software package (Schindelin et al., 2012). For each circRNA construct, silencing-induction was documented by CLSM on more than 5 leaves from independent plants. Note that the final comprehensive statistics of the GFP/YFP silencing induction rate was established by microscopic inspection of all incubated plants for YFP or GFP fluorescence presence.

### Example 2: Production of circular RNAs (circRNAs)

The first step in producing circRNAs involved *in vitro* transcription of a first single chain RNA molecule with a simple loop (SEQ ID NO: 25 for YFP-targeting constructs and SEQ ID NO: 29 for STM-targeting constructs) (see Figure 1A, top), and second single chain RNA molecules (one of SEQ ID NOs: 26-28 for YFP-targeting constructs and SEQ ID NOs: 30-32 for STM-targeting constructs), each with a simple loop (see Figure 1A, top; SEQ ID NO: 26 for T7-YFP; SEQ ID NO: 30 for T7-STM) or a loop comprising either a minimal (SEQ ID NO: 27 for T7-YFP; SEQ ID NO: 31 for T7-STM) or full (SEQ ID NO: 28 for T7-YFP; SEQ ID NO: 32 for T7-STM) TLS structure motif. To produce first and second single chain RNA molecules with a single 5' monophosphate group required to eventually ligate and seal circular RNAs (circRNAs) resulting from hybridization of the first and second single chain RNA molecules, the first and second single chain RNA molecules were first treated with Shrimp Alkaline Phosphatase to remove the 5' triphosphate group incorporated by RNA polymerase, and then subsequently with T4 polynucleotide kinase to restore a 5' monophosphate group to the first and second single chain RNA molecules. These first and second single chain RNA molecules were hybridized to form a dumb-bell shaped circular RNA with either two simple loops (see Figure 1B, top), one simple loop and a minimal TLS motif also known as dDT (see Figure 1B, second from top) or one simple loop and a full TLS motif (see Figure 1B, second from bottom) and two gaps between nucleotides representing the ends of the first and second single chain RNA molecules (nicked circRNA). The complementary sense and antisense strand sequences of the first and second single chain RNA molecules were designed in such a way that following annealing of the sense and antisense sequences, the single nicks in the phosphodiester backbone on each side of the circRNAs were positioned within a stable dsRNA region of the molecule. These two nicks were sealed by RNA ligase reaction to form a covalently closed, single chain circRNA (see Figure 1C, SEQ ID NOs: 33 (two simple loops), 34 (one simple loop and one minimal TLS structure motif), and 35 (one simple loop and one full TLS structure motif) for YFP-targeting constructs and SEQ ID NOs 36 (two simple loops), 37 (one simple loop and one minimal TLS structure motif), and 38 (one simple loop and one full TLS structure motif) for STM-targeting constructs). Furthermore, all the circRNA variants were produced *in vitro* either with and without m⁵C bases, and RNase protection assays were used to confirm their integrity (see Figure 2). The presence of m⁵C bases results in a delayed degradation of single molecule RNA molecules by RNase A (see Figure 2A). Circular RNA is not digested by RNase H (see Figure 2B). The circRNAs were then characterized for RNA stability and topically applied to *A. thaliana* seedlings to evaluate their capacity to induce the silencing of *GFP*/*YFP* transgenes and the endogenous shoot-specific *STM* mRNA*.*

### Example 3: Melting curve analysis of circRNA variants indicates high stability

The stability of topically applied dsRNA most likely plays a substantial role in its ability to be internalized by cells and in triggering gene silencing. Of course, the stability of dsRNA molecules depends on biophysical parameters, such as the extent of base pairing and the 3D-folding structure of the molecule (Jerabek-Willemsen et al., 2011, Assay Drug DevTechnol 9, p.342-353). In particular, the melting temperature serves as an indicator of stability of dsRNA molecules. For this reason, the effect of the TLS motifs and m5C methylation on the melting temperature of circular RNAs (nicked) was determined. In these measurements, the circular RNAs were subjected to an increasing temperature gradient (Mergny and Lacroix, 2003, Methods Enzymol 371, p.13-33; Mergny et al., 2005, Nucleic Acids Res 33, e138), and the adenine analogue 2-aminopurine (2-AP) was used as a probe for RNA conformational dynamics. 2-AP fluoresces under UV light, which is quenched when it base pairs in double stranded regions (Martin et al., 2003, Methods Enzymol 371, p.13-33; Hardman and Thompson, 2006, Biochemistry 45, p.9145-9155). Various in vitro-produced, single chain synthetic GFP/YFP circRNA and 2-AP were subjected to an increasing temperature gradient and the fluorescence changes during thermal denaturation recorded (see Figure 3).

The analysis suggested that melting of YFP circRNAs was complete at the highest temperature (95°C) used (Figure 3a). Notably, single chain circular RNA with two simple loops ("circRNA-2xHP") possessed the highest melting temperature (Tₘ) value (68.86 ± 0.69°C (± S.E.)), which increased significantly to 78.41 ± 0.35°C when m⁵C modified bases were present in single chain circRNA-2xHP molecules. As predicted by the RNA-fold minimal free energy algorithm (Lorenz et al., 2011, Algorithms Mol Biol 6, 26), addition of a minimal TLS motif ("dDT") and a full TLS motif ("TLS") significantly lowered the thermostability of single chain circRNAs. CircRNA-dDT showed a melting point of 63.15 ± 1.02°C, whereas circRNA-TLS with additional stem-loops had a lower Tₘ of 58.38 ± 0.85°C. However, the Tₘ of single chain circRNA-dDT and circRNA-TLS increased to 71.35 ± 0.86°C and to 72.07 ± 0.67°C, respectively, when methylated cytosine bases were incorporated into the circRNAs (see Figure 3b). Overall, the thermophoretic measurements revealed a significant increase in thermostability of m⁵C-methylated single chain circRNAs compared to non-methylated single chain circRNAs, and that circRNA-2xHP was more stable than circRNA-dDT and circRNA-TLS.

### Example 4: Topically applied circular RNAs induce silencing of GFP/YFP transcripts in transgenic lines of Arabidopsis

To evaluate the silencing capacity of the circular RNAs, transgenic *Arabidopsis* seedlings expressing endoplasmic reticulum (ER)-localized GFP (ER-GFP) and peroxisome-localized YFP (PEX11D-YFP) were incubated with nicked circRNAs comprising either two simple loops, one simple loop and dDT, or one simple loop and a full TLS (see schematic depiction in Figure 1B). Expression of GFP in the untreated ER-GFP transgenic line was stable over all developmental stages, whereas the untreated PEX11D-YFP line showed auto-silencing in -10% of 3-5 week-old transgenic plants (see Figure 4 and Table 2).

**Table 2. Number of plants showing PEX11D-YFP silencing after incubation with circRNA.**

| **circRNA construct** | **# plants silenced /# plants treated** |
|---|---|
| mock | 3 / 28 |
| circRNA-2xHP | 7 / 9 |
| circRNA-dDT | 3 / 7 |
| circRNA-TLS | 8 / 11 |

Therefore, the ER-GFP transgenic line was mainly used to test topical application of circRNA variants for induction of transgene silencing. All topical circRNA treatments were done on 5 to 6 Arabidopsis seedlings germinated in 96-well plates containing 100 µL of 0.5x MS agar medium per well. Approximately 5 µg/well of nicked or ligated covalently closed version of various RNA constructs, with and without m⁵C, were added 5 to 7 days after germination (DAG). The seedlings were then transferred two to three days after incubation (dai) with the various RNA constructs onto culture plates to evaluate YFP/GFP silencing by confocal laser scanning microscopy (CLSM) (see Figures 5, 6, 7, and 8).

In line with the reported silencing capacity of ectopically applied RNA molecules, the tested *YFP*/*GFP* ssRNA-HP (second single chain RNA molecule) and all circRNA variants induced transgene silencing in multiple plants within three weeks of treatment (see Figures 4-8; Tables 2-4).

**Table 3. Percentage of plants showing silencing of ER-GFP after RNA incubation.**

| **construct** | % ***ER-GFP* silencing** | | | |
|---|---|---|---|---|
| | **nicked** | | **ligated** | |
| | **C** | **m⁵C** | **C** | **m⁵C** |
| **ssRNA-HP (second single chain RNA molecule)** | **32.2 ± 12.6** | **45.6 ± 19.3** | **-** | **-** |
| **circRNA-2xHP** | **64.6 ± 16.5** | **72.0 ± 10.9** | **67.7 ±14.1** | **73.6 ± 9.8** |
| **circRNA-dDT** | **76.8 ± 13.9** | **89.3 ± 7.6** | **88.9 ± 9.0** | **93.0 ± 5.0** |
| **circRNA-TLS** | **69.0 ± 23.1** | **84.7 ± 13.8** | **84.7 ± 15.0** | **84.5 ± 5.1** |
| **mock control** | **0 (n=60)** | | | |

The mean % GFP ± S.D. is shown in the table (number of biological replicates, n=3; each biological replicate was a plate of 10-15 seedlings incubated with indicated RNA).

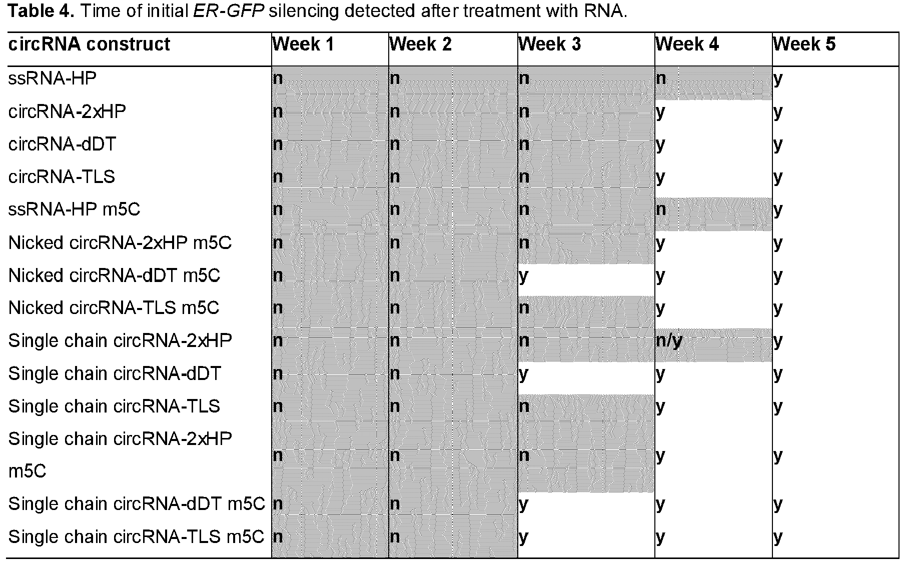

Non-methylated and non-ligated (nicked) ssRNA-HP, circRNA-2xHP, circRNA-dDT, and circRNA-TLS constructs showed a silencing efficiency of 32.9%, 64.6%, 76.8%, and 67.0%, respectively. Methylated and nicked ssRNA-HP, circRNA-2xHP, circRNA-dDT, and circRNA-TLS constructs showed a higher silencing efficiency of 45.6%, 72.0%, 89.8%, and 84.7%, respectively (Tables 3, 5).

**Table 5. Significance values for ER-GFP silencing experiments after treatment with various circRNA obtained by Fisher's exact test using a 2x2 contingency tables (Graphpad Prism Software).**

| Experimental class 1 vs. 2 | No. of Plants Silenced | No. of Plants Not Silenced | p Value (Fisher's exact test) |
|---|---|---|---|
| 1. Treated | 249 | 86 | 0.0001* |
| 2. Mock (Control) | 0 | 60 | |
| | | | |
| 1. All m⁵C-RNAs | 133 | 34 | 0.0332* |
| 2. All RNAs | 116 | 52 | |
| | | | |
| 1. circRNA-single chain | 128 | 31 | 0.2725 |
| 2. circRNA-nicked | 101 | 19 | |
| | | | |
| 1. circRNA-dDT-nicked | 88 | 14 | 0.0026* |
| 2. circRNA-2xHP-nicked | 71 | 33 | |
| | | | |
| 1. circRNA-TLS-nicked | 80 | 22 | 0.1161 |
| 2. circRNA-2xHP-nicked | 71 | 33 | |
| | | | |
| 1. circRNA-dDT-nicked | 88 | 14 | 0.1981 |
| 2. circRNA-TLS-nicked | 80 | 22 | |
| | | | |
| 1. circRNA-2xHP-m⁵C-nicked | 37 | 16 | 0.6799 |
| 2. circRNA-2xHP-nicked | 34 | 19 | |
| | | | |
| 1. circRNA-dDT-m⁵C-nicked | 50 | 5 | 0.2453 |
| 2. circRNA-d DT -nicked | 38 | 8 | |
| | | | |
| 1. circRNA-TLS-m⁵C-nicked | 41 | 8 | 0.2382 |
| 2. circRNA-TLS-nicked | 39 | 14 | |
| | | | |
| 1. circRNA-2xHP-single chain | 37 | 16 | 0.8338 |
| 2. circRNA-2xHP-nicked | 34 | 17 | |
| | | | |
| 1. circRNA-dDT-single chain | 46 | 5 | 0.3888 |
| 2. circRNA-dDT-nicked | 42 | 8 | |
| | | | |
| 1. circRNA-TLS-single chain | 45 | 10 | 0.4701 |
| 2. circRNA-TLS-nicked | 35 | 12 | |

| | | | |
|---|---|---|---|
| *Treatments are found to be statistically significant in this study (showing the two-tailed p value < 0.05). | | | |

Thus, presence of m⁵C nucleotides resulted in a > 10% increase of silenced plants, which was statistically significant (Fishers exact test: p<0.033; Table 5). Of the forms of RNA tested, the non-methylated, non-ligated ssRNA-HP (second single chain RNA molecule) construct that lacks extensive dsRNA regions and harbors a small hairpin at the 3' end was the least effective construct and induced silencing in 32.9% plants, whereas the same methylated ssRNA-HP (second single chain RNA molecule) induced silencing in 45.6% of plants. Thus, incorporation of m⁵C nucleotides also resulted in an > 10% increase of overall silencing efficiency in largely single-stranded single chain RNA constructs lacking long dsRNA regions (as depicted in Figure 1A, top).

The inventors next asked how circularization of the nicked circRNAs by RNA ligation to produce single chain circRNAs might impact the efficiency of *ER-GFP* silencing. Importantly, all topically applied ligated, covalently closed single chain circRNAs resulted in enhanced rates of silencing compared to the respective nicked circRNAs.

The single chain circRNAs without m⁵C base modifications induced silencing in 67.7% (HP), 88.9% (dDT), and 81.2% (TLS) of plants. This increased rate of silencing was further increased by introducing m⁵C bases into the single chain circRNA, with 73.6%, 93.0%, and 84.5% of plants treated with HP, dDT, and TLS methylated single chain circRNAs showing transgene silencing, respectively (see Tables 3, 4). Notably, by combining a dTD pinhead/loop structure, m⁵C bases, and ligation of the circRNA to produce single chain circRNA increased the silencing efficiency to > 90%, which was a three-fold higher induction rate of transgene silencing compared to treatment with the ssRNA-HP control. In line with its high silencing efficiency, all circRNA-dDT constructs also showed the earliest time point of detectable *ER-GFP* gene silencing 2-3 weeks after incubation (see Table 4).

In general, circRNAs harboring the dDT motif were significantly better in inducing gene silencing compared to circRNA-HP constructs (p< 0.003; Students T-Test; see Figure 5c). Methylated and nicked circRNAs were almost as potent as methylated or non-methylated ligated single-chain circRNAs. As seen with methylated vs. non-methylated nicked circRNAs, ligated single chain methylated circRNAs were in general significantly better in inducing gene silencing when compared to nicked and non-methylated circRNAs (p<0.034; Students T-Test). Methylated and nicked circRNAs compared to non-methylated and ligated single chain circular RNAs did not show a significant difference (p> 0.871, Students T-Test) in inducing gene silencing, which suggests that m⁵C methylation as wells as ligation to produce single chain circRNAs improves comparably gene silencing efficiency.

### Example 5: circRNAs silence STM in the apical meristem

To evaluate the effectiveness of circRNAs to silence an endogenous gene, *SHOOT-MERISTEMLESS (STM)* was targeted, which is specifically expressed in the apical shoot meristem (SAM) (Long et al., 1996, Nature 379, p.66-69; Gallois et al., 2002, Development 129, p.3207-3217; Lenhard et al., 2002, Development 129, p.3195-3206). *STM* encodes for a homeodomain transcription factor required for SAM maintenance (Long et al., 1996, Nature 379, p.66-69). *STM* overexpression lines (*GR-STM*) activated by dexamethasome (DEX) show ectopic meristematic tissues, form leaf-like structures lacking trichomes, and are stunted in growth (Gallois et al., 2002 Development 129, p.3207-3217; Lenhard et al., 2002, Development 129, p.3195-3206) (see Figure 9). Thus, the inventors hypothesized that *STM* silencing induced by topically applied *STM* circRNAs should result in normal leaf formation and improved plant growth in GR-STM overexpression lines, and in stunted growth of the non-transgenic wild type.

To test *STM* cricRNAs for inducing silencing of *STM,* non-transgenic wild-type and DEX-treated *GR-STM* lines were incubated with ssRNA-HP (second single chain RNA molecule) and with selected circRNA variants showing lowest and highest *ER-GFP* silencing efficiencies, respectively. As with *ER-GFP* silencing assays, non-transgenic wild-type and *GR-STM* seedlings were incubated with circRNA for 2-3 days and then transferred from 96-well plates to square plates for further phenotypic analysis.

The inventors first addressed the ability of circRNA constructs to silence overexpression of STM in *GR-STM* (Gallois et al., 2002 Development 129, p.3207-3217). As expected, *GR-STM* plants treated with DEX alone (n>50) never formed normal leaves with trichomes, showed no flower formation after 3-4 weeks, and root growth stopped at a very early stage of seedling development (see Figures 6a, 9). When also treated with non-methylated or m⁵C-methylated ssRNA-HP (second single-chain RNA molecule), 11.1 ± 3.7% or 15.9 ± 6.9% of plants, respectively, showed a suppression of the *GR-STM* phenotype, and formed leaves and showed improved root growth (see Figure 9, Table 6). Further, incubation with DEX and nicked or ligated (single chain), methylated *STM* circRNA-dDT resulted in suppression of the *GR-STM* phenotype in 25.2 ± 9.0% and 22.8 ± 4.4%, respectively. Similarly, treatment of *GR-STM* plants with DEX and nicked or ligated (single chain), methylated *STM* circRNA-TLS suppressed the *GR-STM* phenotype in 25.7 ± 1.9% and 26.2 ± 1.7 % of the seedlings, respectively (Table 6). Thus, according to the root and shoot growth and leaf formation, all tested circRNAs were able to suppress expression of *STM* transcripts in *GR-STM* plants. Here, the most effective constructs were methylated nicked or ligated (single chain) circRNA-TLS constructs with a significantly higher gene-silencing rate than circRNA-HP constructs (25.9 ± 1.8% vs. 16.1 ± 4.6%; T-test p<0.001) and with a slightly higher non-significant gene-silencing rate than detected with circRNA-dDT (24.0 ± 7.2%).

**Table 6. Percentage of GR-STM transgenics and wild-type plants showing a STM-related gene silencing phenotype after incubation with RNAs.**

| **construct** | **% *GR-STM* silencing** | | | | % **wild-type *STM* silencing** | | | |
|---|---|---|---|---|---|---|---|---|
| | **nicked** | | **Single chain** | | **nicked** | | **Single chain** | |
| | **C** | **m⁵C** | **C** | **m⁵C** | **C** | **m⁵C** | **C** | **m⁵C** |
| **ssRNA-HP (second single chain RNA molecule)** | **11.1 ± 3.9 (4/36)** | **15.9 ± 6.9 (6/38)** | **-** | **-** | **4.8 ± 3.4 (2/41)** | **7.4 ± 6.3 (3/41)** | **-** | **-** |
| **circRNA- 2xHP** | **14.8 ± 5.6 (6/40)** | **17.5 ± 2.9 (7/40)** | **n/a** | **n/a** | **12.6 ±3.9 (5/40)** | **15.8 ± 7.3 (6/39)** | **n/a** | **n/a** |
| **circRNA-dDT** | **25.2.0 ± 9.0 (9/40)** | **24.3 ± 0.8 (9/37)** | **n/a** | **22.8 ± 4.4 (9/39)** | **18.4 ± 8.3 (7/37)** | **18.8 ± 4.8 (7/38)** | **n/a** | **18.8 ± 4.8 (7/38)** |
| **circRNA-TLS** | **n/a** | **25.7 ± 1.9 (10/39)** | **n/a** | **26.2 ± 1.7 (10/38)** | **n/a** | **11.6 ± 3.8 (5/44)** | **n/a** | **19.4 ± 4.8 (7/42)** |
| **mock control** | **0 (0/50)** | | | | **0 (0/50)** | | | |

The mean % of STM related phenotypes ± S.D. is shown in the table (number of biological replicates, n=3). each biological replicate was a plate of 10-15 seedlings incubated with indicated circRNA. Note that numbers in parentheses represent the total number of plants silenced vs. total number of plants treated with RNA constructs. n/a: not analysed.

Treatment of non-transgenic wild-type (Col-0) plants with *STM* circRNAs resulted in plants showing a phenotype similar to the DEX-induced *GR-STM* plants that was characterized by limited or no shoot and root growth (see Figure 6). Here, nicked or ligated (single chain), methylated STM circRNA-dDT inhibited shoot and root growth in 18.4 ± 8.3% or 18.8 ± 4.8% of non-transgenic wild-type plants, respectively (see Figure 6b, Table 6). Nicked or ligated (single chain), methylated *STM* circRNA-TLS induced an *STM* silencing phenotype in 11.6 ± 3.8 % and 19.5 ± 4.8% of the seedlings, respectively (Table 2). Non-methylated and methylated ssRNA-HP (second single chain RNA molecule) remained the least effective for inducing *STM* silencing with only 4.8 ± 3.4% and 7.4 ± 6.3% of plants, respectively, showing symptoms of *STM* silencing. As seen with treated *GR-STM* plants the most effective constructs were ligated circRNA-TLS (19.4% ± 4.8%) and circRNA-dDT (18.8 ±8%). However, no significant difference was detected between the individual circRNA constructs.

The relatively low rate of circRNA-induced silencing of endogenous *STM* in non-transgenic wild type may be due the fact that *STM* transcripts are only naturally produced in the SAM, which limits the effectiveness of exogenously applied circRNAs to induce silencing. Though, all circRNA constructs were effectively inducing a *STM* silencing phenotype ranging from approx. 11 % to approx. 20 % (see Table 6), which indicates that topically applied circRNAs can trigger silencing of endogenous mRNA, at least in the shoot apical meristem.

### Example 6: Comparison of circular RNAs with two TLS structure motifs with non-circular RNAs lacking TLS structure motifs (open ends)

The inventors further addressed the ability of circRNA constructs to silence overexpression of GUS in *A. thaliana 35S:GUS* transgenic lines used in previous studies to test the silencing efficiency based on nanoclay technology (Mitter et al., 2017, Nat Plants 3, 16207).

### RNA production

PCR primers with T7 promoter and GUS matching sequences corresponding to previously published sequences (Mitter et al., 2017, Nat Plants 3, 16207) were used to amplify cDNA templates (SEQ ID NOs: 39 and 40) as laid out in Table 7 using according plasmid templates (Mitter et al., 2017, Nat Plants 3, 16207).

Sense and antisense single chain RNA molecules were then generated in a single reaction from cDNA templates using T7 RNA polymerase as follows:
1. cDNA template 1 (SEQ ID NO: 39) was used to produce both sense and antisense single chain RNA molecules (SEQ ID NOs: 41 and 42, respectively) in one reaction, which resulted in a double stranded RNA molecule with open ends ("T7-GUS ds"), as previously published (Mitter et al., 2017, Nat Plants 3, 16207).
2. cDNA template 2 (SEQ ID NO: 40) was used to produce both sense and antisense single chain RNA molecules (SEQ ID NOs: 43 and 44, respectively) in one reaction, which resulted in a circular RNA carrying two full TLS structure motifs plus an additional stem loop, in which the open ends of T7-GUS ds are fixed by the additional stem loops (as depicted in Figure 1B, bottom).

Primers are shown in Table 7. One T7 reaction was used to synthesize both sense and antisense single chain RNA molecules using a RiboMax Kit (RiboMAXTM Large Scale RNA Production System-T7 Cat# P1300, Promega) in a single reaction. For *in vitro* synthesis of non-methylated ds RNA molecules, 20 mM of each rNTP was used. For the synthesis of methylated ds RNA molecules, rCTP nucleic bases were replaced with 20 mM m5CTP bases (5-Methylcytidine-5'triphosphate, Cat# N-1014, TriLink Biotechnologies). The quality and quantity of synthesized ds RNA molecules was estimated using both 1% Agarose gel electrophoresis and NanoDrop 2000.

**Table 7.List of primers used to generate cDNA PCR products with T7 sequences to serve as templates for synthesizing in one reaction both sense and antisense ds RNA molecules for circRNAs.**

| **cDNA template** c**reated*** | **Forward PCR primer** | **Reverse PCR primer** |
|---|---|---|
| **T7-GUS ds (1) (SEQ ID No: 39)** | TAA TAC GAC TCA CTA TA GGG GAAAAGTGTACGTATCACCG (SEQ ID NO: 45) | TAA TAC GAC TCA CTA TA GGG AAAGCCAGTAAAGTAGAACG (SEQ ID NO: 46) |
| **T7-GUS-TLS ds (2) (SEQ ID No: 40)** | TATCAGAGCCAGGTTTCGATCC TGGGACCTGTGGGTTATGGGC CCACCACGCTTCCGCTGCGCC ACTCTGATAATGCA TAA TAC GAC TCA CTA TA GGG GAAAAGTGTACGTATCACCG (SEQ ID NO: 47) | TATCAGAGCCAGGTTTCGATCCTG GGACCTGTGGGTTATGGGCCCAC CACGCTTCCGCTGCGCCACTCTGA TAATGCA TAA TAC GAC TCA CTA TA GGG AAAGCCAGTAAAGTAGAACG (SEQ ID NO: 48) |

T7 sequences present in PCR primers is shown in bold letters, TLS structure motif sequences present in PCR primer is shown in italicized letters.

According to quantitative RT-PCR measurements (using primers: AATCAAAAAACTCGACGGCCTGTG (SEQ ID NO: 49) and AACTGCCTGGCACAGCAATTGC (SEQ ID NO: 50) for GUS and primers: CACCACAACAGCAGAGCGGGA (SEQ ID NO: 51) and CCCACAAACGAGGGCTGGAACA (SEQ ID NO: 52) for actin control), *35S:GUS* seedlings treated with T7*-GUS* ds RNA (open ends) in the greenhouse or in controlled environmental chambers showed relatively weak downregulation of GUS RNA presence after 5 days of incubation (Figures 10a and 10b) (n=3 each with ∼10 plants). This was consistent with previous observations showing that dsRNA (open ends) induces an approx. 20% transcript reduction (Mitter et al., 2017, Nat Plants 3, 16207). However, the same transgenic plants treated with methylated T7*-GUS* ds RNA (open ends) or non-methylated or m⁵C-methylated T7-GUS-TLS ds RNA (circular nicked) showed a significant increase of *GUS* RNA silencing (>30%; p < 0.01; n=3 each ∼10 seedlings) compared with T7-GUS ds RNA treatment (see Figure 10 a and b). This indicates that m⁵C methylated RNA and the use of TLS structure motifs significantly increases silencing efficiency. Notably, non-methylated and m⁵C-methylated T7-GUS-TLS ds RNA were equally efficient, showing >50% GUS transcript reduction (see Figure 10 a and b). Thus, methylation, addition of TLS sequences, or both significantly improves silencing of *GUS* transcripts after 5 days of incubation. Again, the most effective constructs were the RNA molecules harboring the full TLS structure motif.

Water, *GFP*

## Claims

1. A single-chain circular RNA comprising:
a. a sense strand sequence,
b. an antisense strand sequence complementary to the sense strand sequence,
c. a first and a second loop sequence, wherein the first loop sequence is located between one end of the sense strand sequence and one end of the antisense strand sequence and wherein the second loop sequence is located between one end of the sense strand sequence and one end of the antisense strand sequence, connecting the sense and antisense strand sequences,
wherein the first and second loop sequences have different or the same sequences, wherein the first and second loop sequences are at least 3 nucleotides in length, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

2. The single-chain circular RNA of claim 1, wherein either the sense strand sequence or the antisense strand sequence contains a nick.

3. A circular RNA consisting of:
a. a first single-chain RNA molecule consisting of:
i. a sense strand sequence,
ii. a first pairing sequence located 3' or 5' of the sense strand sequence,
iii. a loop sequence located 3' or 5' of the first pairing sequence, wherein the loop sequence is at least 3 nucleotides in length, and
iv. a second pairing sequence located 3' or 5' of the loop sequence, wherein the second pairing sequence is complementary to the first pairing sequence,
wherein the first and second pairing sequences are paired to form a stem; and
b. a second single-chain RNA molecule comprising
i. an antisense strand sequence complementary to the sense strand sequence of the first single-chain RNA molecule,
ii. a first pairing sequence located 3' or 5' of the antisense strand sequence,
iii. a loop sequence located 3' or 5' of the antisense strand sequence, wherein the loop sequence is at least 3 nucleotides in length, and
iv. a second pairing sequence located 3' or 5' of the loop sequence, wherein the second pairing sequence is complementary to the first pairing sequence,
wherein the first and second pairing sequences are paired to form a stem;
wherein the locations in the first and second single-chain RNA molecule are all 3' or all 5', wherein the loop sequences of the first and second single-chain RNA molecules have different orthe same sequences, wherein the sense strand sequence and the antisense strand sequence are paired to form a stem, wherein the sense and antisense strand sequences correspond to nucleotide sequences of a target gene or target transcript, and wherein the nucleotide sequences of the target gene or target transcript are complementary to each other.

4. The circular RNA of any one of claims 1-3, wherein the second loop sequence or the loop sequence of the second single-chain RNA molecule is a stem-loop sequence and comprises a tRNA-like sequence (TLS) motif structure, optionally wherein the first loop sequence or the loop sequence of the first single-chain RNA molecule is also a stem-loop sequence and comprises a tRNA-like sequence (TLS) motif structure.

5. The circular RNA of claim 4, wherein the TLS motif structure is
a. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val}, preferably wherein the TLS motif structure is tRNA^{Met}, or
b. selected from the group consisting of tRNA^{Ala}, tRNA^{Arg}, tRNA^{Asn}, tRNA^{Asp}, tRNA^{Cys}, tRNA^{Gln}, tRNA^{Glu}, tRNA^{Gly}, tRNA^{His}, tRNA^{Ile}, tRNA^{Leu}, tRNA^{Lys}, tRNA^{Met}, tRNA^{phe}, tRNA^{Pro}, tRNA^{Ser}, tRNA^{Thr}, tRNA^{Tip}, tRNA^{Tyr}, tRNA^{Val} lacking a dihydrouridine arm and a TψC arm, preferably wherein the TLS motif structure is tRNA^{Met} lacking a dihydrouridine arm and a TψC arm,
c. a viral 3' TLS sequences from a virus forming a tRNA related structure.
d. a viroid forming stem-loop or pseudo-knot structures related to tRNAs
e. a precursor micro RNA or messenger RNA stem-loop structure mediating intercellular RNA transport.

6. The circular RNA of any one of claims 1-5 comprising at least one methylated cytosine (m⁵C) residue, optionally wherein all cytosine residues of the circular RNA are m⁵C residues.

7. The circular RNA of any one of claims 1-6, wherein the stem formed from the sense strand sequence and the antisense strand sequence is 19 base pairs or longer in length.

8. A composition comprising the circular RNA of any one of claims 1-7.

9. The composition of claim 8, wherein the composition is a pharmaceutical composition further comprising a pharmaceutically acceptable carrier.

10. The composition of claim 8, wherein the composition further comprises
a. a permeability-enhancing agent, optionally wherein the permeability-enhancing agent is selected from the group consisting of a surfactant, and organic solvent, an aqueous solution of an organic solvent, an oxidizing agent, an acid, a base, an oil, an enzyme, or a combination thereof; or
b. an adjuvant; or
c. a peptide-carrier; or
d. a vesicular endocytosis-carrier; or
e. a micro-carrier; or
f. a nano -carrier.

11. A kit comprising the circular RNA of any one of claims 1-7 or the composition of any one of claims 8-10.

12. A method of producing a circular RNA, comprising the steps of:
a. synthesizing a first single-chain RNA molecule comprising:
i. a sense strand sequence,
ii. a first pairing sequence located 3' or 5' of the sense strand sequence,
iii. a loop sequence located 3' or 5' of the first pairing sequence, wherein the loop sequence is at least 3 nucleotides in length, optionally wherein the loop sequence is a stem-loop sequence and comprises a tRNA-like sequence (TLS) motif structure, and
iv. a second pairing sequence located 3' or 5' of the loop sequence, wherein the second pairing sequence is complementary to the first pairing sequence,
wherein the first and second pairing sequences are paired to form a stem;
b. synthesizing a second single-chain RNA molecule comprising
i. an antisense strand sequence complementary to the sense strand sequence of the first single-chain RNA molecule,
ii. a first pairing sequence located 3' or 5' of the antisense strand sequence,
iii. a loop sequence located 3' or 5' of the antisense strand sequence, wherein the loop sequence is at least 3 nucleotides in length, optionally wherein the loop sequence is a stem-loop sequence and comprises a tRNA-like sequence (TLS) motif structure, and
iv. a second pairing sequence located 3' or 5' of the loop sequence, wherein the second pairing sequence is complementary to the first pairing sequence,
wherein the first and second pairing sequences are paired to form a stem;
c. hybridizing the first and second single-chain RNA molecules such that the sense and antisense strand sequences pair to form a stem, thereby producing a double-chain circular RNA; and
d. optionally ligating the hybridized first and second single-chain RNA molecules of step c to form a single-chain circular RNA,
wherein the locations in the first and second single-chain RNA molecule are all 3' or all 5', wherein the loop sequence of the first and the loop sequence of the second single-chain RNA molecules have different or the same sequences, wherein the sense and antisense strand sequences correspond to nucleotide sequences of a target gene or target transcript, and wherein the nucleotide sequences of the target gene and or transcript are complementary to each other.

13. A method of producing a circular RNA, comprising the steps of:
a. synthesizing a single-chain RNA molecule comprising:
i. a sense strand sequence,
ii. a first pairing sequence located 3' or 5' of the sense strand sequence,
iii. a first loop sequence located 3' or 5' of the first pairing sequence, wherein the first loop sequence is at least 3 nucleotides in length, and
iv. a second pairing sequence located 3' or 5' of the first loop sequence, wherein the second pairing sequence is complementary to the first pairing sequence,
v. an antisense strand sequence complementary to the sense strand sequence, located 3' or 5' of the second pairing sequence; and
vi. a loop sequence located 3' or 5' of the antisense strand sequence, optionally wherein the second loop sequence comprises a tRNA-like sequence (TLS) motif structure, and
wherein the locations are all 3' or all 5',
b. hybridizing the single-chain RNA molecule such that the sense and antisense strand sequences pair to form a stem and the first and second pairing sequences from a stem, thereby producing a circular single-chain RNA; and
c. optionally ligating the hybridized circular single-chain RNA molecules of step b,
wherein the first and second loop sequences have different or the same sequences, wherein the sense and antisense strand sequences correspond to nucleotide sequences of a target gene or target transcript, and wherein the nucleotide sequences of the target gene and or transcript are complementary to each other.

14. The method of claim 12 or 13,
wherein in steps a and b 5-methylcytidine-5'triphosphate is provided; or
wherein the circular RNA comprises a methylation motif and the method further comprises exposing the circular RNA to a methyltransferase enzyme modifying cytosine (C) to 5-methylcytosine (m5C) RNA in vivo, ex vivo, or in vitro

15. A method of inhibiting the expression of a target gene or the function of a target transcript in a cell comprising contacting the cell with the circular RNA of any one of claims 1-7 or the composition of any one of claims 8-10 in an amount sufficient to inhibit expression of the target gene or the function of the target transcript, wherein the sense and antisense sequences of the circular RNA correspond to nucleotide sequences of the target gene or transcript, wherein the nucleotide sequences of the target gene or transcript are complementary to each other.

16. The method of claim 15, wherein the method is performed *in vitro, ex vivo,* or *in vivo.*

17. The method of claim 15 or claim 16, wherein the cell is a cell selected from the group consisting of a eukaryotic cell, optionally wherein the cell is an animal cell, a fungal cell, or a plant cell.

18. The method of any one of claims 15-17, wherein the target gene or target transcript is a gene or transcript selected from the group of an endogenous gene or transcript, a transgene, or a gene or transcript of a pathogen, optionally wherein the pathogen is selected from the group consisting of a viroid, a virus, a bacterium, a fungus, and a parasitic animal or plant.

19. The method of any one of claims 15-18, wherein the cell is present in an organism, and wherein the circular RNA or composition is introduced within a body cavity of the organism inside or outside the cell.

20. The method of any one of claims 15-18, wherein the cell is present in an organism, and wherein the circular RNA or composition is introduced into the organism by extracellular injection.

21. The method of any one of claims 15-18, wherein the cell is present in an organism, and wherein the circular RNA or composition is introduced into the organism by feeding.

22. The method of any one of claims 15-18, wherein the cell is a plant cell present in a plant, and wherein the circular RNA or composition is topically applied to the surface of the plant, optionally wherein the circular RNA or composition is applied by soaking, coating, spraying, biolistically introducing, electroporating or high-pressure spraying.

23. The method of any one of claims 15-18, wherein the cell is a plant cell present in a plant, and wherein the circular RNA or composition is introduced into the plant by applying the circular RNA or composition on plant tissue after cutting the plant or on callus tissue formed after cutting the plant, optionally wherein the plant is a grape vine.

24. The method of any one of claims 15-23, wherein the expression of the target gene or function of the target transcript is inhibited by at least 10%, 15%, 20%, 25%, 30%, 35%, 40%, 45%, 50%, 55%, 60%, 65%, 70%, 75%, 80%, 85%, 90%, 95%, 96%, 97%, 98%, or 99%.

25. The method of any one of claims 15-24, wherein inhibiting the expression of a target gene or the function of a target transcript results in suppression of a pathogen.

26. The method of any one of claims 15-24, wherein the expression of the target gene or the function of the target transcript is dysregulated in the cell and wherein inhibiting the expression of a target gene or function of the target transcript results in better regulation.

27. The circular RNA of any one of claims 1-7 or the composition of any one of claims 8-10 for use in a method of treating a condition in a subject in need thereof, wherein the condition is selected from the group consisting of an infectious disease, feeding animals, a heritable disease, cancer, a disease involving the dysregulation of the expression of a gene, and a disease involving dysregulation of the function of a transcript.
